(19) Europäisches Patentamt / European Patent Office / Office européen des brevets

(11) **EP 3 831 961 B1**

(12) **EUROPEAN PATENT SPECIFICATION**

(45) Date of publication and mention
of the grant of the patent:
**03.09.2025 Bulletin 2025/36**

(21) Application number: **19843453.2**

(22) Date of filing: **29.07.2019**

(51) International Patent Classification (IPC):
**G16B 20/00** (2019.01)    **G16B 25/10** (2019.01)
**G16B 25/20** (2019.01)

(52) Cooperative Patent Classification (CPC):
(C-Sets available)
**G16B 25/20; C12Q 1/6841; C12Q 1/6886;
G16B 20/00; G16B 25/10;** C12Q 2600/112;
C12Q 2600/154; C12Q 2600/158; G16B 20/10
(Cont.)

(86) International application number:
**PCT/CN2019/098209**

(87) International publication number:
**WO 2020/024911 (06.02.2020 Gazette 2020/06)**

(54) **GRADING MODEL FOR DETECTING BENIGN AND MALIGNANT DEGREES OF THYROID TUMORS, AND APPLICATION THEREOF**

EINSTUFUNGSMODELL ZUM NACHWEIS VON GUTARTIGEN UND BÖSARTIGEN SCHILDRÜSENTUMOREN UND SEINE VERWENDUNG

MODÈLE DE CLASSIFICATION POUR LA DÉTECTION DU CARACTÈRE BÉNIN OU MALIN DES TUMEURS DE LA THYROÏDE ET SON APPLICATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **01.08.2018   CN 201810860667**

(43) Date of publication of application:
**09.06.2021 Bulletin 2021/23**

(73) Proprietor: **Lisen Imprinting Diagnostics Wuxi
Co., Ltd
Wuxi City, Jiangsu 214000 (CN)**

(72) Inventors:
• **CHENG, Tong**
**Wuxi, Jiangsu 214000 (CN)**
• **ZHOU, Ning**
**Wuxi, Jiangsu 214000 (CN)**

(74) Representative: **Grund, Martin et al
Grund Intellectual Property Group
Patentanwälte und Solicitor PartG mbB
Steinsdorfstraße 2
80538 München (DE)**

(56) References cited:
WO-A1-2007/015993    WO-A1-2016/115530
CN-A- 101 152 575    CN-A- 101 553 576
CN-A- 105 018 585    CN-A- 108 315 424
CN-A- 108 929 851    US-A1- 2010 124 747

• **QING ZHONG ET AL: "Image-based
computational quantification and visualization of
genetic alterations and tumour heterogeneity",
SCIENTIFIC REPORTS, vol. 6, no. 1, 1 April 2016
(2016-04-01), pages 24146, XP055667022, DOI:
10.1038/srep24146**

**(Cont. next page)**

- **SARQUIS, M.S. ET AL.: "High Frequency of Loss of Heterozygosity in Imprinted, Compared with Nonimprinted , Genomic Regions in Follicular Thyroid Carcinomas and Atypical Adenomas", JOURNAL OF CLINICAL ENDOCRINOLOGY AND METABOLISM, vol. 91, no. 1, 1 January 2006 (2006-01-01), pages 262 - 269, XP055683805, ISSN: 0021-972X, DOI: 10.1210/jc.2005-1880**
- **XING, MINGZHAO: "Minireview: Gene Methylation in Thyroid Tumorigenesis", ENDOCRINOLOGY, vol. 148, no. 3, 1 March 2007 (2007-03-01), pages 948 - 953, XP055683798, ISSN: 0013-7227, DOI: 10.1210/en.2006-0927**

(52) Cooperative Patent Classification (CPC): (Cont.)

C-Sets
**C12Q 1/6841, C12Q 2537/143, C12Q 2537/164, C12Q 2537/165**

## Description

## Technical Field

The present disclosure pertains to the field of biotechnology, such as the field of genetic diagnosis, such as a grading model and its applications, such as a grading model for detecting the degree of benignity/malignancy of a thyroid tumor and its applications, such as a grading model for detecting the degree of benignity/malignancy of a thyroid tumor through a combination of imprinted genes and a device using the model.

## Description of Related Art

According to statistics of the World Health Organization (WHO), the year 2012 saw 298,100 new cases of thyroid cancer worldwide, including about 90,000 newly diagnosed cases in China and about 25,520 in the United States. Most thyroid cancers are differentiated to a relatively high degree and have a very good prognosis, with a five-year survival rate higher than 95%. Some thyroid cancers, however, tend to metastasize as early as their existence as a tiny tumor. Poorly differentiated thyroid cancer can be lethal and has a median survival time ranging from four months to five years. Currently, difficulties in the pathological diagnosis of thyroid cancer include the discrimination between follicular thyroid adenomas (FTA), which are benign, and follicular thyroid carcinomas (FTC), which are malignant, and the determination of the benignity or malignancy of Hürthle cell tumors (HCT). The BRAF gene mutation tests are the only applicable detection method nowadays but can only be used to detect papillary thyroid cancer; they do not work when it comes to the diagnosis of FTC and HCT. In China, the morbidity of thyroid cancer increased 4.6-fold in the last decade and is still on the rise, with about 100 thousand new cases each year, of which about 10% are the difficult-to-diagnose FTC and HCT. These ambiguous cases and three to four times as many suspicious cases amount to approximately 40-50 thousand cases that require definite diagnosis each year. When patients in other countries are taken into account, the total will exceed 100,000. It is therefore imperative to develop a detection technique that is more accurate than the existing ones.

According to conventional pathology, a cell is diagnosed as benign or malignant based on the size, morphology, and invasiveness of the cell and the relationship between the cell and the surrounding cells or tissue. As this approach imposes substantial limitations on the discovery of early changes in a cell (cancer), methods for diagnosing cancer on a cellular or molecular level were once a hot research topic. With continuous in-depth studies in molecular biology, more and more molecular detection techniques have been applied to the diagnosis of cancer.

Cancer takes place when cells grow and/or divide in an uncontrolled manner resulting from an accumulation of epigenetic changes and genetic variations over time. In a conventional pathological diagnosis, a thyroid tumor is determined to be benign or malignant based on the variations in size, morphology, and structure of cells and tissue. With the development and advancement of molecular biology, more and more molecular detection techniques have been used to detect thyroid cancer. Analyses of the process of cancer development have shown that molecular changes (in epigenetics and genetics) occur far earlier than variations in cell morphology and tissue structure, and this is why molecular biology-based detection is more sensitive than other detection methods in detecting cancer at an early stage.

WO 2007/015993 A1 discloses comparing the methylation status/percentage in a potentially diseased sample to a control sample in order to detect genomic imprinting disorders such as Prader-Willi Syndrome and Angelman Syndrome. WO2016115530 furthermore describes a method for evaluating cancer by detecting epigenetic changes.

According to the above, the existing thyroid cancer diagnosis methods are in need of a new detection system and detection model that can be applied to a patient's biopsy samples to analyze thyroid cancer-related changes in a molecular marker on a cellular level, in order to provide more accurate prognosis and diagnosis information.

## Brief Summary of the Invention

The invention is defined by the appended claims.

The present disclosure provides an imprinted gene grading model for detecting the degree of benignity/malignancy of a thyroid tumor, a diagnostic method using the model, and other applications of the model.

To achieve the foregoing objective, the following technical solution is used:

In one aspect, the present invention relates to an imprinted gene grading model applicable to a thyroid tumor, the model being configured to calculate changes in the following expressed quantities of an imprinted gene in the thyroid tumor and grade an expression state of the imprinted gene accordingly: a total expressed quantity of the imprinted gene, an expressed quantity of the imprinted gene with a loss of imprinting, and an expressed quantity of the imprinted gene with a copy number variation;

wherein the imprinted gene is any one, or a combination of at least two, of Z1, Z11, and Z16, with the imprinted gene Z1 being Gnas, the imprinted gene Z11 being Grb10, and the imprinted gene Z16 being Snrpn/Snurf;

wherein the total expressed quantity of the imprinted gene, the expressed quantity of the imprinted gene with a loss of imprinting, and the expressed quantity of the imprinted gene with a copy number variation are calculated by the following formulas:

$$\text{the total expressed quantity of the imprinted gene} = (b+c+d)/(a+b+c+d) \times 100\%;$$

$$\text{an expressed quantity of the imprinted gene being normal} = b/(b+c+d) \times 100\%;$$

the expressed quantity of the imprinted gene with a loss of imprinting (LOI) = $c/(b+c+d) \times 100\%$; and

the expressed quantity of the imprinted gene with a copy number variation (CNV) = $d/(b+c+d) \times 100\%$;

where a is the number of cells that, after being stained with hematoxylin, show no mark in the nucleus of each said cell, meaning the imprinted gene is not expressed in the nucleus of each said cell; b is the number of cells that, after being stained with hematoxylin, show one red/brown mark in the nucleus of each said cell, meaning the imprinted gene is present in the nucleus of each said cell; c is the number of cells that, after being stained with hematoxylin, show two red/brown marks in the nucleus of each said cell, meaning the imprinted gene in the nucleus of each said cell is affected by a loss of imprinting; and d is the number of cells that, after being stained with hematoxylin, show more than two red/brown marks in the nucleus of each said cell, meaning the imprinted gene in the nucleus of each said cell has a copy number variation; and
wherein the expressed quantity of the imprinted gene with a loss of imprinting, the expressed quantity of the imprinted gene with a copy number variation, and the total expressed quantity of the imprinted gene are each graded as one of five different grades;
the expressed quantity of each of the imprinted genes Z1 and Z16 with a loss of imprinting, the expressed quantity of each of the imprinted genes Z1 and Z16 with a copy number variation, and the total expressed quantity of each of the imprinted genes Z1 and Z16 are graded into the following five grades:

Grade 0: applicable when any one or at least two of the following conditions are met: the expressed quantity of one of the imprinted genes Z1 and Z16 with a loss of imprinting is less than 12%, the expressed quantity of one of the imprinted genes Z1 and Z16 with a copy number variation is less than 1%, and the total expressed quantity of one of the imprinted genes Z1 and Z16 is less than 25%;
Grade I: applicable when any one or at least two of the following conditions are met: the expressed quantity of one of the imprinted genes Z1 and Z16 with a loss of imprinting is 12%-20%, the expressed quantity of one of the imprinted genes Z1 and Z16 with a copy number variation is 1%-2%, and the total expressed quantity of one of the imprinted genes Z1 and Z16 is 25%-35%;
Grade II: applicable when any one or at least two of the following conditions are met: the expressed quantity of one of the imprinted genes Z1 and Z16 with a loss of imprinting is 20%-25%, the expressed quantity of one of the imprinted genes Z1 and Z16 with a copy number variation is 2%-3%, and the total expressed quantity of one of the imprinted genes Z1 and Z16 is 35%-45%;
Grade III: applicable when any one or at least two of the following conditions are met: the expressed quantity of one of the imprinted genes Z1 and Z16 with a loss of imprinting is 25%-30%, the expressed quantity of one of the imprinted genes Z1 and Z16 with a copy number variation is 3%-5%, and the total expressed quantity of one of the imprinted genes Z1 and Z16 is 45%-60%; and
Grade IV: applicable when any one or at least two of the following conditions are met: the expressed quantity of one of the imprinted genes Z1 and Z16 with a loss of imprinting is greater than 30%, the expressed quantity of one of the imprinted genes Z1 and Z16 with a copy number variation is greater than 5%, and the total expressed quantity of one of the imprinted genes Z1 and Z16 is greater than 60%; and

the expressed quantity of the imprinted gene Z11 with a loss of imprinting, the expressed quantity of the imprinted gene Z11 with a copy number variation, and the total expressed quantity of the imprinted gene Z11 are graded into the following five grades:

Grade 0: applicable when any one or at least two of the following conditions are met: the expressed quantity of the imprinted gene Z11 with a loss of imprinting is less than 10%, the expressed quantity of the imprinted gene Z11 with a copy number variation is less than 0.5%, and the total expressed quantity of the imprinted gene Z11 is less than 15%;

Grade I: applicable when any one or at least two of the following conditions are met: the expressed quantity of the imprinted gene Z11 with a loss of imprinting is 10%-15%, the expressed quantity of the imprinted gene Z11 with a copy number variation is 0.5%-1%, and the total expressed quantity of the imprinted gene Z11 is 15%-20%;

Grade II: applicable when any one or at least two of the following conditions are met: the expressed quantity of the imprinted gene Z11 with a loss of imprinting is 15%-20%, the expressed quantity of the imprinted gene Z11 with a copy number variation is 1%-2%, and the total expressed quantity of the imprinted gene Z11 is 20%-30%;

Grade III: applicable when any one or at least two of the following conditions are met: the expressed quantity of the imprinted gene Z11 with a loss of imprinting is 20%-25%, the expressed quantity of the imprinted gene Z11 with a copy number variation is 2%-3%, and the total expressed quantity of the imprinted gene Z11 is 30%-40%; and

Grade IV: applicable when any one or at least two of the following conditions are met: the expressed quantity of the imprinted gene Z11 with a loss of imprinting is greater than 25%, the expressed quantity of the imprinted gene Z11 with a copy number variation is greater than 3%, and the total expressed quantity of the imprinted gene Z11 is greater than 40%.

In an embodiment, the model calculates the expressed quantities of any one of the imprinted genes Z1, Z11, and Z16, preferably the imprinted gene Z1 or Z16, and more preferably the imprinted gene Z1. In another embodiment, the model calculates the expressed quantities of a combination of any two of the imprinted genes Z1, Z11, and Z16, preferably a combination of the imprinted genes Z1 and Z16 or a combination of the imprinted genes Z1 and Z11. In another embodiment, the imprinted gene further comprises any one, or a combination of at least two, of Z3, Z4, Z5, Z6, Z8, Z10, and Z13, with the imprinted gene Z3 being Peg10, the imprinted gene Z4 being Igf2r, the imprinted gene Z5 being Mest, the imprinted gene Z6 being Plagl1, the imprinted gene Z8 being Dcn, the imprinted gene Z10 being Gatm, and the imprinted gene Z13 being Sgce;

the expressed quantity of each of the imprinted genes Z3, Z4, Z5, Z6, Z8, Z10, and Z13 with a loss of imprinting, the expressed quantity of each of the imprinted genes Z3, Z4, Z5, Z6, Z8, Z10, and Z13 with a copy number variation, and the total expressed quantity of each of the imprinted genes Z3, Z4, Z5, Z6, Z8, Z10, and Z13 are graded into the following five grades:

Grade 0: applicable when any one or at least two of the following conditions are met: the expressed quantity of one of the imprinted genes Z3, Z4, Z5, Z6, Z8, Z10, and Z13 with a loss of imprinting is less than 10%, the expressed quantity of one of the imprinted genes Z3, Z4, Z5, Z6, Z8, Z10, and Z13 with a copy number variation is less than 0.5%, and the total expressed quantity of one of the imprinted genes Z3, Z4, Z5, Z6, Z8, Z10, and Z13 is less than 15%;

Grade I: applicable when any one or at least two of the following conditions are met: the expressed quantity of one of the imprinted genes Z3, Z4, Z5, Z6, Z8, Z10, and Z13 with a loss of imprinting is 10%-15%, the expressed quantity of one of the imprinted genes Z3, Z4, Z5, Z6, Z8, Z10, and Z13 with a copy number variation is 0.5%-1%, and the total expressed quantity of one of the imprinted genes Z3, Z4, Z5, Z6, Z8, Z10, and Z13 is 15%-20%;

Grade II: applicable when any one or at least two of the following conditions are met: the expressed quantity of one of the imprinted genes Z3, Z4, Z5, Z6, Z8, Z10, and Z13 with a loss of imprinting is 15%-20%, the expressed quantity of one of the imprinted genes Z3, Z4, Z5, Z6, Z8, Z10, and Z13 with a copy number variation is 1%-2%, and the total expressed quantity of one of the imprinted genes Z3, Z4, Z5, Z6, Z8, Z10, and Z13 is 20%-30%;

Grade III: applicable when any one or at least two of the following conditions are met: the expressed quantity of one of the imprinted genes Z3, Z4, Z5, Z6, Z8, Z10, and Z13 with a loss of imprinting is 20%-25%, the expressed quantity of one of the imprinted genes Z3, Z4, Z5, Z6, Z8, Z10, and Z13 with a copy number variation is 2%-3%, and the total expressed quantity of one of the imprinted genes Z3, Z4, Z5, Z6, Z8, Z10, and Z13 is 30%-40%; and

Grade IV: applicable when any one or at least two of the following conditions are met: the expressed quantity of one of the imprinted genes Z3, Z4, Z5, Z6, Z8, Z10, and Z13 with a loss of imprinting is greater than 25%, the expressed quantity of one of the imprinted genes Z3, Z4, Z5, Z6, Z8, Z10, and Z13 with a copy number variation is greater than 3%, and the total expressed quantity of one of the imprinted genes Z3, Z4, Z5, Z6, Z8, Z10, and Z13 is greater than 40%. In an embodiment, the model calculates the expressed quantities of a combination of the ten imprinted genes Z1, Z3, Z4, Z5, Z6, Z8, Z10, Z11, Z13, and Z16.

In another aspect, the present invention relates to a device for detecting a degree of benignity/malignancy of a thyroid tumor, wherein the device uses the model as described above and comprises:

(1) a sampling unit for obtaining a test sample;

(2) a probe designing unit for designing a primer specific to the sequence of the imprinted gene, wherein the primer serves as a probe;

(3) a detection unit for performing in-situ hybridization between the probe designed by the probe designing unit and the test sample; and

(4) an analysis unit for analyzing expression of the imprinted gene through microscopic imaging;

wherein the analysis unit calculates and grades, via the model of any of claims 1-5, the expressed quantity of the imprinted gene with a loss of imprinting, the expressed quantity of the imprinted gene with a copy number variation, and the total expressed quantity of the imprinted gene, and diagnoses the degree of benignity/malignancy of the thyroid tumor according to grading results of the expressed quantities of the imprinted gene with, respectively, a loss of imprinting and a copy number variation.

In another aspect, the present invention relates to a method for detecting a degree of benignity/malignancy of a thyroid tumor, wherein the method uses the model of any of claims 1-5 or the device of claim 6 and comprises the steps of:

(1) obtaining a test sample;
(2) designing a primer specific to the sequence of the imprinted gene, wherein the primer serves as a probe;
(3) performing in-situ hybridization between the probe designed in step (2) and the test sample; and
(4) analyzing expression of the imprinted gene through microscopic imaging so as to diagnose the degree of benignity/malignancy of the thyroid tumor;

wherein step (4) comprises calculating and grading, via the model of any of claims 1-5, the expressed quantity of the imprinted gene with a loss of imprinting, the expressed quantity of the imprinted gene with a copy number variation, and the total expressed quantity of the imprinted gene, and diagnosing the degree of benignity/malignancy of the thyroid tumor according to grading results of the expressed quantities of the imprinted gene with, respectively, a loss of imprinting and a copy number variation;
optionally wherein the test sample in step (1) is tissue and/or cells obtained from a human, optionally wherein the test sample is a paraffin section of a tissue and/or a cell smear prepared by a thyroid needle biopsy.

In am embodiment, the in-situ hybridization uses an RNAscope in-situ hybridization method, optionally wherein the RNAscope in-situ hybridization method uses a single- or multiple-channel chromogenic reagent kit or a single- or multiple-channel fluorescent reagent kit, preferably a single-channel red/brown chromogenic reagent kit or a multiple-channel fluorescent reagent kit. In another embodiment, the degree of benignity/malignancy of the thyroid tumor is classified as one of the following: benign tumor, potential thyroid cancer, early-stage thyroid cancer, intermediate-stage thyroid cancer, and advanced thyroid cancer. In another embodiment, the degree of benignity/malignancy of the thyroid tumor is diagnosed as benign tumor when the expressed quantities of each of the imprinted genes Z1, Z3, Z4, Z5, Z6, Z8, Z10, Z11, Z13, and Z16 with, respectively, a loss of imprinting and a copy number variation are both graded as Grade 0; or when the expressed quantity of not more than one of the imprinted genes Z1, Z3, Z4, Z5, Z6, Z8, Z10, Z11, Z13, and Z16 with a loss of imprinting is graded as Grade I, and the expressed quantity of not more than one of the imprinted genes Z1, Z3, Z4, Z5, Z6, Z8, Z10, Z11, Z13, and Z16 with a copy number variation is graded as Grade I;

the degree of benignity/malignancy of the thyroid tumor is diagnosed as potential thyroid cancer when the expressed quantities of at least two of the imprinted genes Z1, Z3, Z4, Z5, Z6, Z8, Z10, Z11, Z13, and Z16 with a loss of imprinting are graded as Grade I; or when the expressed quantities of at least two of the imprinted genes Z1, Z3, Z4, Z5, Z6, Z8, Z10, Z11, Z13, and Z16 with a copy number variation are graded as Grade I; or when the expressed quantity of not more than one of the imprinted genes Z1, Z3, Z4, Z5, Z6, Z8, Z10, Z11, Z13, and Z16 with a loss of imprinting is graded as Grade II, and the expressed quantity of not more than one of the imprinted genes Z1, Z3, Z4, Z5, Z6, Z8, Z10, Z11, Z13, and Z16 with a copy number variation is graded as Grade II;
the degree of benignity/malignancy of the thyroid tumor is diagnosed as early-stage thyroid cancer when the expressed quantities of at least two of the imprinted genes Z1, Z3, Z4, Z5, Z6, Z8, Z10, Z11, Z13, and Z16 with a loss of imprinting are graded as Grade II; or when the expressed quantities of at least two of the imprinted genes Z1, Z3, Z4, Z5, Z6, Z8, Z10, Z11, Z13, and Z16 with a copy number variation are graded as Grade II; or when the expressed quantity of not more than one of the imprinted genes Z1, Z3, Z4, Z5, Z6, Z8, Z10, Z11, Z13, and Z16 with a loss of imprinting is graded as Grade III, and the expressed quantity of not more than one of the imprinted genes Z1, Z3, Z4, Z5, Z6, Z8, Z10, Z11, Z13, and Z16 with a copy number variation is graded as Grade III;
the degree of benignity/malignancy of the thyroid tumor is diagnosed as intermediate-stage thyroid cancer when the expressed quantities of at least two of the imprinted genes Z1, Z3, Z4, Z5, Z6, Z8, Z10, Z11, Z13, and Z16 with a loss of imprinting are graded as Grade III; or when the expressed quantities of at least two of the imprinted genes Z1, Z3, Z4, Z5, Z6, Z8, Z10, Z11, Z13, and Z16 with a copy number variation are graded as Grade III; or when the expressed quantity of not more than one of the imprinted genes Z1, Z3, Z4, Z5, Z6, Z8, Z10, Z11, Z13, and Z16 with a loss of imprinting is graded as Grade IV, and the expressed quantity of not more than one of the imprinted genes Z1, Z3, Z4, Z5, Z6, Z8, Z10, Z11, Z13, and Z16 with a copy number variation is graded as Grade IV; and

the degree of benignity/malignancy of the thyroid tumor is diagnosed as advanced thyroid cancer when the expressed quantities of at least two of the imprinted genes Z1, Z3, Z4, Z5, Z6, Z8, Z10, Z11, Z13, and Z16 with a loss of imprinting are graded as Grade IV; or when the expressed quantities of at least two of the imprinted genes Z1, Z3, Z4, Z5, Z6, Z8, Z10, Z11, Z13, and Z16 with a copy number variation are graded as Grade IV.

In another aspect, the present invention relates to a use of the model and/or the device as described above in detecting a thyroid tumor. In an embodiment, a degree of benignity/malignancy of the thyroid tumor is classified as one of the following: benign tumor, potential thyroid cancer, early-stage thyroid cancer, intermediate-stage thyroid cancer, and advanced thyroid cancer. In another embodiment, a degree of benignity/malignancy of the thyroid tumor is diagnosed as benign tumor when the expressed quantities of each of the imprinted genes Z1, Z3, Z4, Z5, Z6, Z8, Z10, Z11, Z13, and Z16 with, respectively, a loss of imprinting and a copy number variation are both graded as Grade 0; or when the expressed quantity of not more than one of the imprinted genes Z1, Z3, Z4, Z5, Z6, Z8, Z10, Z11, Z13, and Z16 with a loss of imprinting is graded as Grade I, and the expressed quantity of not more than one of the imprinted genes Z1, Z3, Z4, Z5, Z6, Z8, Z10, Z11, Z13, and Z16 with a copy number variation is graded as Grade I;

the degree of benignity/malignancy of the thyroid tumor is diagnosed as potential thyroid cancer when the expressed quantities of at least two of the imprinted genes Z1, Z3, Z4, Z5, Z6, Z8, Z10, Z11, Z13, and Z16 with a loss of imprinting are graded as Grade I; or when the expressed quantities of at least two of the imprinted genes Z1, Z3, Z4, Z5, Z6, Z8, Z10, Z11, Z13, and Z16 with a copy number variation are graded as Grade I; or when the expressed quantity of not more than one of the imprinted genes Z1, Z3, Z4, Z5, Z6, Z8, Z10, Z11, Z13, and Z16 with a loss of imprinting is graded as Grade II, and the expressed quantity of not more than one of the imprinted genes Z1, Z3, Z4, Z5, Z6, Z8, Z10, Z11, Z13, and Z16 with a copy number variation is graded as Grade II;

the degree of benignity/malignancy of the thyroid tumor is diagnosed as early-stage thyroid cancer when the expressed quantities of at least two of the imprinted genes Z1, Z3, Z4, Z5, Z6, Z8, Z10, Z11, Z13, and Z16 with a loss of imprinting are graded as Grade II; or when the expressed quantities of at least two of the imprinted genes Z1, Z3, Z4, Z5, Z6, Z8, Z10, Z11, Z13, and Z16 with a copy number variation are graded as Grade II; or when the expressed quantity of not more than one of the imprinted genes Z1, Z3, Z4, Z5, Z6, Z8, Z10, Z11, Z13, and Z16 with a loss of imprinting is graded as Grade III, and the expressed quantity of not more than one of the imprinted genes Z1, Z3, Z4, Z5, Z6, Z8, Z10, Z11, Z13, and Z16 with a copy number variation is graded as Grade III;

the degree of benignity/malignancy of the thyroid tumor is diagnosed as intermediate-stage thyroid cancer when the expressed quantities of at least two of the imprinted genes Z1, Z3, Z4, Z5, Z6, Z8, Z10, Z11, Z13, and Z16 with a loss of imprinting are graded as Grade III; or when the expressed quantities of at least two of the imprinted genes Z1, Z3, Z4, Z5, Z6, Z8, Z10, Z11, Z13, and Z16 with a copy number variation are graded as Grade III; or when the expressed quantity of not more than one of the imprinted genes Z1, Z3, Z4, Z5, Z6, Z8, Z10, Z11, Z13, and Z16 with a loss of imprinting is graded as Grade IV, and the expressed quantity of not more than one of the imprinted genes Z1, Z3, Z4, Z5, Z6, Z8, Z10, Z11, Z13, and Z16 with a copy number variation is graded as Grade IV; and

the degree of benignity/malignancy of the thyroid tumor is diagnosed as advanced thyroid cancer when the expressed quantities of at least two of the imprinted genes Z1, Z3, Z4, Z5, Z6, Z8, Z10, Z11, Z13, and Z16 with a loss of imprinting are graded as Grade IV; or when the expressed quantities of at least two of the imprinted genes Z1, Z3, Z4, Z5, Z6, Z8, Z10, Z11, Z13, and Z16 with a copy number variation are graded as Grade IV.

The present disclosure provides an imprinted gene grading model that is applicable to a thyroid tumor. The model calculates changes in the following expressed quantities of an imprinted gene in a thyroid tumor and grades the expression state of the imprinted gene accordingly: a total expressed quantity of the imprinted gene, an expressed quantity of the imprinted gene with a loss of imprinting, and an expressed quantity of the imprinted gene with a copy number variation.

The imprinted gene is any one, or a combination of at least two, of Z1, Z11, and Z16, wherein the imprinted gene Z1 is Gnas, the imprinted gene Z11 is Grb10, and the imprinted gene Z16 is Snrpn/Snurf.

Loss of imprinting refers to the activation (demethylation) of a previously silenced allele of an imprinted gene and is the most common and earliest epigenetic change in cancer and therefore a characteristic that can be used as a pathological marker. By contrast, loss of imprinting seldom occurs in a healthy cell.

The inventor of the present disclosure has found that a diagnostic sensitivity of at least 64.3% can be achieved for the diagnosis of thyroid tumors by calculating the following expressed quantities of any one of the imprinted genes Z1, Z11, and Z16 in a thyroid tumor: the expressed quantity corresponding to a loss of imprinting and the expressed quantity corresponding to a copy number variation.

In one embodiment of the present disclosure, any one of the imprinted genes Z1, Z11, and Z16 can be detected if only one imprinted gene is to be detected in preliminary detection.

In one embodiment of the present disclosure, either one of the imprinted genes Z1 and Z11 can be detected if only one imprinted gene is to be detected in preliminary detection.

In one embodiment of the present disclosure, the imprinted gene Z1 can be detected if only one imprinted gene is to be detected in preliminary detection.

The inventor has found in one embodiment of the present disclosure that a diagnostic sensitivity of 84.8% can be achieved for the diagnosis of thyroid tumors by detecting only the imprinted gene Z1, 66.7% by detecting only the imprinted gene Z11, and 64.3% by detecting only the imprinted gene Z16.

In one embodiment of the present disclosure, the model calculates the aforesaid expressed quantities of the to-be-detected imprinted gene in such a way that if a combination of two imprinted genes are to be detected, the combination may include any two of Z1, Z11, and Z16, preferably a combination of Z1 and Z11 or a combination of Z1 and Z16.

The inventor has found that diagnostic sensitivity can be increased by calculating the respective total expressed quantities of two or more imprinted genes, the expressed quantities of the two or more imprinted genes with a loss of imprinting, and the expressed quantities of the two or more imprinted genes with a copy number variation. A diagnostic sensitivity of at least 84.6% can be achieved for the diagnosis of thyroid cancer by detecting a combination of any two of the imprinted genes Z1, Z11, and Z16; at least 92.9% by detecting a combination of Z1 and Z16; and at least 97.5% by detecting a combination of Z11 and Z11.

The inventor has found that detecting one, or a combination of at least two, of Z3, Z4, Z5, Z6, Z8, Z10, and Z13 in addition to one, or a combination of at least two, of Z1, Z11, and Z16 for a joint diagnosis not only helps increase the accuracy of detection, but also prevents false positive results thanks to the assistance of additional probes in making the diagnosis, thereby further enhancing the accuracy of detection and allowing all the thyroid tumor samples under examination to be accurately graded and judged.

In one embodiment of the present disclosure, the model calculates the aforesaid expressed quantities of the to-be-detected imprinted gene by calculating the aforesaid expressed quantities of a combination of imprinted genes, namely a combination of the imprinted genes Z1, Z3, Z4, Z5, Z6, Z8, Z10, Z11, Z13, and Z16.

An imprinted gene is determined as having a loss of imprinting when two red/brown marks are present in the nucleus of the cell after the cell is stained with hematoxylin. An imprinted gene is determined as having a copy number variation when more than two red/brown marks are present in the nucleus of the cell after the cell is stained with hematoxylin. A copy number variation occurs when abnormal gene duplication takes place in a cancer cell such that the duplicated gene is expressed as having three or more copies of the original gene.

The following formulas are used to calculate the total expressed quantity of an imprinted gene, the expressed quantity of the imprinted gene with a loss of imprinting, and the expressed quantity of the imprinted gene with a copy number variation:

$$\text{total expressed quantity of the imprinted gene} = (b+c+d)/(a+b+c+d) \times 100\%;$$

$$\text{expressed quantity of the imprinted gene being normal} = b/(b+c+d) \times 100\%;$$

$$\text{expressed quantity of the imprinted gene with a loss of imprinting (LOI)} = c/(b+c+d) \times 100\%;$$

and

$$\text{expressed quantity of the imprinted gene with a copy number variation (CNV)} = d/(b+c+d) \times 100\%;$$

where a is the number of cells that, after being stained with hematoxylin, show no mark in the nucleus, meaning the imprinted gene is not expressed in the nucleus; b is the number of cells that, after being stained with hematoxylin, show one red/brown mark in the nucleus, meaning the imprinted gene is present in the nucleus; c is the number of cells that, after being stained with hematoxylin, show two red/brown marks in the nucleus, meaning the imprinted gene in the nucleus is affected by a loss of imprinting; and d is the number of cells that, after being stained with hematoxylin, show more than two red/brown marks in the nucleus, meaning the imprinted gene in the nucleus has a copy number variation.

In one embodiment of the present disclosure, the hematoxylin-stained marks are selected from but not limited to red marks and brown marks. Staining/marking in other colors is also feasible when calculating the total expressed quantity of an imprinted gene, the expressed quantity of the imprinted gene with a loss of imprinting, and the expressed quantity of the imprinted gene with a copy number variation.

In one embodiment of the present disclosure, in-situ hybridization is carried out by way of a probe, and the nuclei of cells are stained with hematoxylin for signal amplification. Each nucleus is then detected under a 40× or 60× microscope for the presence of the to-be-detected imprinted gene, for any loss of imprinting in the imprinted gene, and for any copy number variation in the imprinted gene. After that, the degree of benignity/malignancy of the tumor sample in question is determined by calculating the expressed quantity of the imprinted gene with a loss of imprinting and the expressed quantity of the imprinted gene with a copy number variation. As the section under detection is merely 10 μm thick, about 20% of the nuclei seen under the microscope are incomplete; in other words, the detection results may be partly false negative.

The total expressed quantity of an imprinted gene, the expressed quantity of the imprinted gene with a loss of imprinting, and the expressed quantity of the imprinted gene with a copy number variation are graded into five different grades. The foregoing expressed quantities of each of ten imprinted genes, namely Z1, Z3, Z4, Z5, Z6, Z8, Z10, Z11, Z13, and Z16, are obtained by counting at least 1200 cells in an area of a sample where the corresponding probe is the most clearly expressed, and the expressed quantities obtained are classified according to the five-grade scale.

The expressed quantities of the imprinted genes Z1 and Z16 with a loss of imprinting, the expressed quantities of the imprinted genes Z1 and Z16 with a copy number variation, and the respective total expressed quantities of the imprinted genes Z1 and Z16 are classified into the following five grades:

Grade 0: applicable when any one or at least two of the following conditions are met: the expressed quantity of the imprinted gene Z1 or Z16 with a loss of imprinting is less than 12%, the expressed quantity of the imprinted gene Z1 or Z16 with a copy number variation is less than 1%, and the total expressed quantity of the imprinted gene Z1 or Z16 is less than 25%;

Grade I: applicable when any one or at least two of the following conditions are met: the expressed quantity of the imprinted gene Z1 or Z16 with a loss of imprinting is 12%-20%, the expressed quantity of the imprinted gene Z1 or Z16 with a copy number variation is 1%-2%, and the total expressed quantity of the imprinted gene Z1 or Z16 is 25%-35%;

Grade II: applicable when any one or at least two of the following conditions are met: the expressed quantity of the imprinted gene Z1 or Z16 with a loss of imprinting is 20%-25%, the expressed quantity of the imprinted gene Z1 or Z16 with a copy number variation is 2%-3%, and the total expressed quantity of the imprinted gene Z1 or Z16 is 35%-45%;

Grade III: applicable when any one or at least two of the following conditions are met: the expressed quantity of the imprinted gene Z1 or Z16 with a loss of imprinting is 25%-30%, the expressed quantity of the imprinted gene Z1 or Z16 with a copy number variation is 3%-5%, and the total expressed quantity of the imprinted gene Z1 or Z16 is 45%-60%; and

Grade IV: applicable when any one or at least two of the following conditions are met: the expressed quantity of the imprinted gene Z1 or Z16 with a loss of imprinting is greater than 30%, the expressed quantity of the imprinted gene Z1 or Z16 with a copy number variation is greater than 5%, and the total expressed quantity of the imprinted gene Z1 or Z16 is greater than 60%;

wherein the aforesaid expressed quantities of either one of the imprinted genes Z1 and Z16 are independent of those of the other.

In one embodiment of the present disclosure, the expressed quantities of the imprinted genes Z3, Z4, Z5, Z6, Z8, Z10, Z11, and Z13 with a loss of imprinting, the expressed quantities of the imprinted genes Z3, Z4, Z5, Z6, Z8, Z10, Z11, and Z13 with a copy number variation, and the respective total expressed quantities of the imprinted genes Z3, Z4, Z5, Z6, Z8, Z10, Z11, and Z13 are classified into the following five grades:

Grade 0: applicable when any one or at least two of the following conditions are met: the expressed quantity of the imprinted gene Z3, Z4, Z5, Z6, Z8, Z10, Z11, or Z13 with a loss of imprinting is less than 10%, the expressed quantity of the imprinted gene Z3, Z4, Z5, Z6, Z8, Z10, Z11, or Z13 with a copy number variation is less than 0.5%, and the total expressed quantity of the imprinted gene Z3, Z4, Z5, Z6, Z8, Z10, Z11, or Z13 is less than 15%;

Grade I: applicable when any one or at least two of the following conditions are met: the expressed quantity of the imprinted gene Z3, Z4, Z5, Z6, Z8, Z10, Z11, or Z13 with a loss of imprinting is 10%-15%, the expressed quantity of the imprinted gene Z3, Z4, Z5, Z6, Z8, Z10, Z11, or Z13 with a copy number variation is 0.5%-1%, and the total expressed quantity of the imprinted gene Z3, Z4, Z5, Z6, Z8, Z10, Z11, or Z13 is 15%-20%;

Grade II: applicable when any one or at least two of the following conditions are met: the expressed quantity of the imprinted gene Z3, Z4, Z5, Z6, Z8, Z10, Z11, or Z13 with a loss of imprinting is 15%-20%, the expressed quantity of the imprinted gene Z3, Z4, Z5, Z6, Z8, Z10, Z11, or Z13 with a copy number variation is 1%-2%, and the total expressed quantity of the imprinted gene Z3, Z4, Z5, Z6, Z8, Z10, Z11, or Z13 is 20%-30%;

Grade III: applicable when any one or at least two of the following conditions are met: the expressed quantity of the imprinted gene Z3, Z4, Z5, Z6, Z8, Z10, Z11, or Z13 with a loss of imprinting is 20%-25%, the expressed quantity of the imprinted gene Z3, Z4, Z5, Z6, Z8, Z10, Z11, or Z13 with a copy number variation is 2%-3%, and the total expressed quantity of the imprinted gene Z3, Z4, Z5, Z6, Z8, Z10, Z11, or Z13 is 30%-40%; and

Grade IV: applicable when any one or at least two of the following conditions are met: the expressed quantity of the imprinted gene Z3, Z4, Z5, Z6, Z8, Z10, Z11, or Z13 with a loss of imprinting is greater than 25%, the expressed quantity of the imprinted gene Z3, Z4, Z5, Z6, Z8, Z10, Z11, or Z13 with a copy number variation is greater than 3%, and the total expressed quantity of the imprinted gene Z3, Z4, Z5, Z6, Z8, Z10, Z11, or Z13 is greater than 40%;

wherein the aforesaid expressed quantities of any one of the imprinted genes Z3, Z4, Z5, Z6, Z8, Z10, Z11, and Z13 are independent of those of another.

In another aspect of the present disclosure, a device for detecting the degree of benignity/malignancy of a thyroid tumor is provided. The device uses the model described above and includes the following units:

(1) a sampling unit for obtaining a test sample;
(2) a probe designing unit for designing a primer, or probe, specific to the sequence of the imprinted gene to be detected;
(3) a detection unit for performing in-situ hybridization between the probe designed by unit (2) and the test sample; and
(4) an analysis unit for analyzing the expression of the imprinted gene through microscopic imaging;
wherein the analysis unit calculates and grades, via the foregoing model, the total expressed quantity of the imprinted gene, the expressed quantity of the imprinted gene with a loss of imprinting, and the expressed quantity of the imprinted gene with a copy number variation, and determines the degree of benignity/malignancy of the thyroid tumor in question according to the grades of the expressed quantities of the imprinted gene with, respectively, a loss of imprinting and a copy number variation.

An imprinted gene is determined as having a loss of imprinting when two red/brown marks are present in the nucleus of the cell after the cell is stained with hematoxylin. An imprinted gene is determined as having a copy number variation when more than two red/brown marks are present in the nucleus of the cell after the cell is stained with hematoxylin. A copy number variation occurs when abnormal gene duplication takes place in a cancer cell such that the duplicated gene is expressed as having three or more copies of the original gene.

The hematoxylin-stained marks are selected from but not limited to red marks and brown marks. Staining/marking in other colors is also feasible when calculating the total expressed quantity of an imprinted gene, the expressed quantity of the imprinted gene with a loss of imprinting, and the expressed quantity of the imprinted gene with a copy number variation.

The detection device can be used to intuitively observe, on a cellular or tissue level and as early as possible, the changes of an imprinted gene in a thyroid tumor so as to determine the degree of benignity/malignancy of the tumor, thereby allowing a patient with an early-stage thyroid tumor to have the best chance of treatment.

In another aspect of the disclosure, a method for detecting the degree of benignity/malignancy of a thyroid tumor is provided. The method uses the foregoing model or device and includes the following steps:

(1) obtaining a test sample;
(2) designing a primer, or probe, specific to the sequence of the imprinted gene to be detected;
(3) performing in-situ hybridization between the probe in step (2) and the test sample; and
(4) analyzing the expression of the imprinted gene through microscopic imaging so as to diagnose the degree of benignity/malignancy of the thyroid tumor in question;

wherein step (4) includes calculating and grading, via the foregoing model, the expressed quantity of the imprinted gene with a loss of imprinting, the expressed quantity of the imprinted gene with a copy number variation, and the total expressed quantity of the imprinted gene, and diagnosing the degree of benignity/malignancy of the thyroid tumor according to the grades of the expressed quantities of the imprinted gene with, respectively, a loss of imprinting and a copy number variation.

In one embodiment of the present disclosure, the test sample in step (1) is tissue and/or cells obtained from a human.

The test sample can be chosen by a person skilled in the art according to practical needs and without limitation, provided that the RNA in the test sample is fixed in time. The test sample stated herein may include either one or both of a paraffin section of a tissue and a cell smear prepared by a thyroid needle biopsy.

The steps of obtaining a paraffin section of a tissue include obtaining a human tumor tissue sample, fixing the tissue with 10% neutral buffered formalin in time, embedding the tissue in paraffin, cutting a 10 $\mu$m-thick section out of the paraffin-embedded tissue, and making a tissue slide with a positively charged slide. As the section is only 10 $\mu$m thick, some of the nuclei seen under a microscope are incomplete, which leads to partly false negative results regarding gene deletion.

The steps of preparing a cell smear by a thyroid needle biopsy include obtaining human cells with a biopsy needle and fixing the cells with 10% neutral buffered formalin in time.

As an experiment sample, a needle biopsy sample has its special advantage not only because the obtainment of the sample causes little harm to the patient and can be done with ease, but also because a needle biopsy allows the exact location of the sample to be known, which is not the case with the drawing of blood, which circulates through the entire body.

In one embodiment of the present disclosure, the test sample is a cell smear prepared by a thyroid needle biopsy.

In one embodiment of the present disclosure, the imprinted genes to be detected are Z1, Z3, Z4, Z5, Z6, Z8, Z10, Z11, Z13, and Z16, wherein the imprinted gene Z1 is Gnas, the imprinted gene Z3 is Peg10, the imprinted gene Z4 is Igf2r, the imprinted gene Z5 is Mest, the imprinted gene Z6 is Plagl1, the imprinted gene Z8 is Dcn, the imprinted gene Z10 is Gatm, the imprinted gene Z11 is Grb10, the imprinted gene Z13 is Sgce, and the imprinted gene Z16 is Snrpn/Snurf.

The imprinted genes Z1(Gnas), Z3(Peg10), Z4(Igf2r), Z5(Mest), Z6(Plagl1), Z8(Dcn), Z10(Gatm), Z11(Grb10), Z13(Sgce), and Z16(Snrpn/Snurf) are expressed to different degrees in a normal tumor cell or tissue, and their expressed quantities and imprinted states change significantly when the tumor cell or tissue turns malignant.

The probe is designed according to the imprinted gene to be detected, namely Z1, Z3, Z4, Z5, Z6, Z8, Z10, Z11, Z13, or Z16, i.e., Gnas, Peg10, Igf2r, Mest, Plagl1, Dcn, Gatm, Grb10, Sgce, or Snrpn/Snurf. More specifically, a sequence is selected from the intron of each of the aforesaid genes as the corresponding probe. The probes used in the embodiments described further below were designed by Advanced Cell Diagnostics.

In one embodiment of the present disclosure, the in-situ hybridization uses an RNAscope in-situ hybridization method.

In one embodiment of the present disclosure, the RNAscope in-situ hybridization method uses a single- or multiple-channel chromogenic reagent kit or a single- or multiple-channel fluorescent reagent kit, preferably a single-channel red/brown chromogenic reagent kit or a multiple-channel fluorescent reagent kit.

The multiple-channel chromogenic reagent kit or multiple-channel fluorescent reagent kit includes a two (or more)-channel chromogenic or fluorescent reagent kit. The two-channel chromogenic reagent kit or multiple-channel fluorescent reagent kit can use two imprinted gene probes to detect the joint expression of each corresponding imprinted gene and another gene or use more than two imprinted gene probes to detect the joint expression of each corresponding imprinted gene and a non-imprinted gene.

The following formulas are used to calculate the total expressed quantity of an imprinted gene, the expressed quantity of the imprinted gene with a loss of imprinting, and the expressed quantity of the imprinted gene with a copy number variation:

$$\text{total expressed quantity of the imprinted gene} = (b+c+d)/(a+b+c+d) \times 100\%;$$

$$\text{expressed quantity of the imprinted gene being normal} = b/(b+c+d) \times 100\%;$$

$$\text{expressed quantity of the imprinted gene with a loss of imprinting (LOI)} = c/(b+c+d) \times 100\%;$$

and

expressed quantity of the imprinted gene with a copy number variation (CNV) = $d/(b+c+d) \times 100\%$;

where a is the number of cells that, after being stained with hematoxylin, show no mark in the nucleus, meaning the imprinted gene is not expressed in the nucleus; b is the number of cells that, after being stained with hematoxylin, show one red/brown mark in the nucleus, meaning the imprinted gene is present in the nucleus; c is the number of cells that, after being stained with hematoxylin, show two red/brown marks in the nucleus, meaning the imprinted gene in the nucleus is affected by a loss of imprinting; and d is the number of cells that, after being stained with hematoxylin, show more than two red/brown marks in the nucleus, meaning the imprinted gene in the nucleus has a copy number variation.

The hematoxylin-stained marks are selected from but not limited to red marks and brown marks. Staining/marking in other colors is also feasible when calculating the total expressed quantity of an imprinted gene, the expressed quantity of the imprinted gene with a loss of imprinting, and the expressed quantity of the imprinted gene with a copy number variation.

The in-situ hybridization is carried out by way of the probe. The nuclei of cells are stained with hematoxylin for signal amplification. Each nucleus is detected under a 40× or 60× microscope for the presence of the to-be-detected imprinted gene, for any loss of imprinting in the imprinted gene, and for any copy number variation in the imprinted gene. The degree of benignity/malignancy of the tumor sample in question is determined by calculating the total expressed quantity of the imprinted gene, the expressed quantity of the imprinted gene with a loss of imprinting, and the expressed quantity of the imprinted gene with a copy number variation. As the section under detection is merely 10 μm thick, about 20% of the nuclei seen under the microscope are incomplete; in other words, the detection results may be partly false negative.

The expressed quantity of the to-be-detected imprinted gene with a loss of imprinting, the expressed quantity of the imprinted gene with a copy number variation, and the total expressed quantity of the imprinted gene are graded into five different grades.

More specifically, the foregoing expressed quantities of each of ten imprinted genes, namely Z1, Z3, Z4, Z5, Z6, Z8, Z10, Z11, Z13, and Z16, are obtained by counting at least 1200 cells in an area of a sample where the corresponding probe is the most clearly expressed, and the expressed quantities obtained are classified according to the five-grade scale.

The expressed quantities of the imprinted genes Z1 and Z16 with a loss of imprinting, the expressed quantities of the imprinted genes Z1 and Z16 with a copy number variation, and the respective total expressed quantities of the imprinted genes Z1 and Z16 are classified into the following five grades:

Grade 0: applicable when any one or at least two of the following conditions are met: the expressed quantity of the imprinted gene Z1 or Z16 with a loss of imprinting is less than 12%, the expressed quantity of the imprinted gene Z1 or Z16 with a copy number variation is less than 1%, and the total expressed quantity of the imprinted gene Z1 or Z16 is less than 25%;

Grade I: applicable when any one or at least two of the following conditions are met: the expressed quantity of the imprinted gene Z1 or Z16 with a loss of imprinting is 12%-20%, the expressed quantity of the imprinted gene Z1 or Z16 with a copy number variation is 1%-2%, and the total expressed quantity of the imprinted gene Z1 or Z16 is 25%-35%;

Grade II: applicable when any one or at least two of the following conditions are met: the expressed quantity of the imprinted gene Z1 or Z16 with a loss of imprinting is 20%-25%, the expressed quantity of the imprinted gene Z1 or Z16 with a copy number variation is 2%-3%, and the total expressed quantity of the imprinted gene Z1 or Z16 is 35%-45%;

Grade III: applicable when any one or at least two of the following conditions are met: the expressed quantity of the imprinted gene Z1 or Z16 with a loss of imprinting is 25%-30%, the expressed quantity of the imprinted gene Z1 or Z16 with a copy number variation is 3%-5%, and the total expressed quantity of the imprinted gene Z1 or Z16 is 45%-60%; and

Grade IV: applicable when any one or at least two of the following conditions are met: the expressed quantity of the imprinted gene Z1 or Z16 with a loss of imprinting is greater than 30%, the expressed quantity of the imprinted gene Z1 or Z16 with a copy number variation is greater than 5%, and the total expressed quantity of the imprinted gene Z1 or Z16 is greater than 60%;

wherein the aforesaid expressed quantities of either one of the imprinted genes Z1 and Z16 are independent of those of the other.

The expressed quantities of the imprinted genes Z3, Z4, Z5, Z6, Z8, Z10, Z11, and Z13 with a loss of imprinting, the expressed quantities of the imprinted genes Z3, Z4, Z5, Z6, Z8, Z10, Z11, and Z13 with a copy number variation, and the respective total expressed quantities of the imprinted genes Z3, Z4, Z5, Z6, Z8, Z10, Z11, and Z13 are classified into the following five grades:

Grade 0: applicable when any one or at least two of the following conditions are met: the expressed quantity of the imprinted gene Z3, Z4, Z5, Z6, Z8, Z10, Z11, or Z13 with a loss of imprinting is less than 10%, the expressed quantity of the imprinted gene Z3, Z4, Z5, Z6, Z8, Z10, Z11, or Z13 with a copy number variation is less than 0.5%, and the total expressed quantity of the imprinted gene Z3, Z4, Z5, Z6, Z8, Z10, Z11, or Z13 is less than 15%;

Grade I: applicable when any one or at least two of the following conditions are met: the expressed quantity of the imprinted gene Z3, Z4, Z5, Z6, Z8, Z10, Z11, or Z13 with a loss of imprinting is 10%-15%, the expressed quantity of the imprinted gene Z3, Z4, Z5, Z6, Z8, Z10, Z11, or Z13 with a copy number variation is 0.5%-1%, and the total expressed quantity of the imprinted gene Z3, Z4, Z5, Z6, Z8, Z10, Z11, or Z13 is 15%-20%;

Grade II: applicable when any one or at least two of the following conditions are met: the expressed quantity of the imprinted gene Z3, Z4, Z5, Z6, Z8, Z10, Z11, or Z13 with a loss of imprinting is 15%-20%, the expressed quantity of the imprinted gene Z3, Z4, Z5, Z6, Z8, Z10, Z11, or Z13 with a copy number variation is 1%-2%, and the total expressed quantity of the imprinted gene Z3, Z4, Z5, Z6, Z8, Z10, Z11, or Z13 is 20%-30%;

Grade III: applicable when any one or at least two of the following conditions are met: the expressed quantity of the imprinted gene Z3, Z4, Z5, Z6, Z8, Z10, Z11, or Z13 with a loss of imprinting is 20%-25%, the expressed quantity of the imprinted gene Z3, Z4, Z5, Z6, Z8, Z10, Z11, or Z13 with a copy number variation is 2%-3%, and the total expressed quantity of the imprinted gene Z3, Z4, Z5, Z6, Z8, Z10, Z11, or Z13 is 30%-40%; and

Grade IV: applicable when any one or at least two of the following conditions are met: the expressed quantity of the imprinted gene Z3, Z4, Z5, Z6, Z8, Z10, Z11, or Z13 with a loss of imprinting is greater than 25%, the expressed quantity of the imprinted gene Z3, Z4, Z5, Z6, Z8, Z10, Z11, or Z13 with a copy number variation is greater than 3%, and the total expressed quantity of the imprinted gene Z3, Z4, Z5, Z6, Z8, Z10, Z11, or Z13 is greater than 40%;

wherein the aforesaid expressed quantities of any one of the imprinted genes Z3, Z4, Z5, Z6, Z8, Z10, Z11, and Z13 are independent of those of another.

In one embodiment of the present disclosure, the degree of benignity/malignancy of a thyroid tumor is classified as one of the following: benign tumor, potential thyroid cancer, early-stage thyroid cancer, intermediate-stage thyroid cancer, and advanced thyroid cancer.

In one embodiment of the present disclosure, the degree of benignity/malignancy of a thyroid tumor is determined as benign tumor when the expressed quantities of each of the imprinted genes Z1, Z3, Z4, Z5, Z6, Z8, Z10, Z11, Z13, and Z16 with, respectively, a loss of imprinting and a copy number variation are both graded as Grade 0; or when the expressed quantity of not more than one of the imprinted genes Z1, Z3, Z4, Z5, Z6, Z8, Z10, Z11, Z13, and Z16 with a loss of imprinting is graded as Grade I, and the expressed quantity of not more than one of the imprinted genes Z1, Z3, Z4, Z5, Z6, Z8, Z10, Z11, Z13, and Z16 with a copy number variation is graded as Grade I;

the degree of benignity/malignancy of the thyroid tumor is determined as potential thyroid cancer when the expressed quantities of at least two of the imprinted genes Z1, Z3, Z4, Z5, Z6, Z8, Z10, Z11, Z13, and Z16 with a loss of imprinting are graded as Grade I; or when the expressed quantities of at least two of the imprinted genes Z1, Z3, Z4, Z5, Z6, Z8, Z10, Z11, Z13, and Z16 with a copy number variation are graded as Grade I; or when the expressed quantity of not more than one of the imprinted genes Z1, Z3, Z4, Z5, Z6, Z8, Z10, Z11, Z13, and Z16 with a loss of imprinting is graded as Grade II, and the expressed quantity of not more than one of the imprinted genes Z1, Z3, Z4, Z5, Z6, Z8, Z10, Z11, Z13, and Z16 with a copy number variation is graded as Grade II;

the degree of benignity/malignancy of the thyroid tumor is determined as early-stage thyroid cancer when the expressed quantities of at least two of the imprinted genes Z1, Z3, Z4, Z5, Z6, Z8, Z10, Z11, Z13, and Z16 with a loss of imprinting are graded as Grade II; or when the expressed quantities of at least two of the imprinted genes Z1, Z3, Z4, Z5, Z6, Z8, Z10, Z11, Z13, and Z16 with a copy number variation are graded as Grade II; or when the expressed quantity of not more than one of the imprinted genes Z1, Z3, Z4, Z5, Z6, Z8, Z10, Z11, Z13, and Z16 with a loss of imprinting is graded as Grade III, and the expressed quantity of not more than one of the imprinted genes Z1, Z3, Z4, Z5, Z6, Z8, Z10, Z11, Z13, and Z16 with a copy number variation is graded as Grade III;

the degree of benignity/malignancy of the thyroid tumor is determined as intermediate-stage thyroid cancer when the expressed quantities of at least two of the imprinted genes Z1, Z3, Z4, Z5, Z6, Z8, Z10, Z11, Z13, and Z16 with a loss of imprinting are graded as Grade III; or when the expressed quantities of at least two of the imprinted genes Z1, Z3, Z4, Z5, Z6, Z8, Z10, Z11, Z13, and Z16 with a copy number variation are graded as Grade III; or when the expressed quantity of not more than one of the imprinted genes Z1, Z3, Z4, Z5, Z6, Z8, Z10, Z11, Z13, and Z16 with a loss of imprinting is graded as Grade IV, and the expressed quantity of not more than one of the imprinted genes Z1, Z3, Z4, Z5, Z6, Z8, Z10, Z11, Z13, and Z16 with a copy number variation is graded as Grade IV; and

the degree of benignity/malignancy of the thyroid tumor is determined as advanced thyroid cancer when the expressed quantities of at least two of the imprinted genes Z1, Z3, Z4, Z5, Z6, Z8, Z10, Z11, Z13, and Z16 with a loss of imprinting are graded as Grade IV; or when the expressed quantities of at least two of the imprinted genes Z1, Z3, Z4, Z5, Z6, Z8, Z10, Z11, Z13, and Z16 with a copy number variation are graded as Grade IV.

In another aspect of the present disclosure, a use of the foregoing model or of the foregoing device in detecting a thyroid tumor is provided.

In one embodiment of the present disclosure, the degree of benignity/malignancy of a thyroid tumor is classified as one of the following: benign tumor, potential thyroid cancer, early-stage thyroid cancer, intermediate-stage thyroid cancer, and advanced thyroid cancer.

In one embodiment of the present disclosure, the degree of benignity/malignancy of a thyroid tumor is determined as benign tumor when the expressed quantities of each of the imprinted genes Z1, Z3, Z4, Z5, Z6, Z8, Z10, Z11, Z13, and Z16 with, respectively, a loss of imprinting and a copy number variation are both graded as Grade 0; or when the expressed quantity of not more than one of the imprinted genes Z1, Z3, Z4, Z5, Z6, Z8, Z10, Z11, Z13, and Z16 with a loss of imprinting is graded as Grade I, and the expressed quantity of not more than one of the imprinted genes Z1, Z3, Z4, Z5, Z6, Z8, Z10, Z11, Z13, and Z16 with a copy number variation is graded as Grade I;

the degree of benignity/malignancy of the thyroid tumor is determined as potential thyroid cancer when the expressed quantities of at least two of the imprinted genes Z1, Z3, Z4, Z5, Z6, Z8, Z10, Z11, Z13, and Z16 with a loss of imprinting are graded as Grade I; or when the expressed quantities of at least two of the imprinted genes Z1, Z3, Z4, Z5, Z6, Z8, Z10, Z11, Z13, and Z16 with a copy number variation are graded as Grade I; or when the expressed quantity of not more than one of the imprinted genes Z1, Z3, Z4, Z5, Z6, Z8, Z10, Z11, Z13, and Z16 with a loss of imprinting is graded as Grade II, and the expressed quantity of not more than one of the imprinted genes Z1, Z3, Z4, Z5, Z6, Z8, Z10, Z11, Z13, and Z16 with a copy number variation is graded as Grade II;

the degree of benignity/malignancy of the thyroid tumor is determined as early-stage thyroid cancer when the expressed quantities of at least two of the imprinted genes Z1, Z3, Z4, Z5, Z6, Z8, Z10, Z11, Z13, and Z16 with a loss of imprinting are graded as Grade II; or when the expressed quantities of at least two of the imprinted genes Z1, Z3, Z4, Z5, Z6, Z8, Z10, Z11, Z13, and Z16 with a copy number variation are graded as Grade II; or when the expressed quantity of not more than one of the imprinted genes Z1, Z3, Z4, Z5, Z6, Z8, Z10, Z11, Z13, and Z16 with a loss of imprinting is graded as Grade III, and the expressed quantity of not more than one of the imprinted genes Z1, Z3, Z4, Z5, Z6, Z8, Z10, Z11, Z13, and Z16 with a copy number variation is graded as Grade III;

the degree of benignity/malignancy of the thyroid tumor is determined as intermediate-stage thyroid cancer when the expressed quantities of at least two of the imprinted genes Z1, Z3, Z4, Z5, Z6, Z8, Z10, Z11, Z13, and Z16 with a loss of imprinting are graded as Grade III; or when the expressed quantities of at least two of the imprinted genes Z1, Z3, Z4, Z5, Z6, Z8, Z10, Z11, Z13, and Z16 with a copy number variation are graded as Grade III; or when the expressed quantity of not more than one of the imprinted genes Z1, Z3, Z4, Z5, Z6, Z8, Z10, Z11, Z13, and Z16 with a loss of imprinting is graded as Grade IV, and the expressed quantity of not more than one of the imprinted genes Z1, Z3, Z4, Z5, Z6, Z8, Z10, Z11, Z13, and Z16 with a copy number variation is graded as Grade IV; and

the degree of benignity/malignancy of the thyroid tumor is determined as advanced thyroid cancer when the expressed quantities of at least two of the imprinted genes Z1, Z3, Z4, Z5, Z6, Z8, Z10, Z11, Z13, and Z16 with a loss of imprinting are graded as Grade IV; or when the expressed quantities of at least two of the imprinted genes Z1, Z3, Z4, Z5, Z6, Z8, Z10, Z11, Z13, and Z16 with a copy number variation are graded as Grade IV.

Compared with the related art, the embodiments disclosed herein use the foregoing detection model or device to enable intuitive observation of the expression of an imprinted gene in a sample taken from a patient with a thyroid tumor. By labeling the imprinted gene in situ, changes in the imprinted gene can be objectively, intuitively, and precisely detected at an early stage. Not only that, a quantitative model is provided. Thus, the present disclosure contributes greatly to diagnoses in molecular pathology.

## Brief Description of the Several Views of the Drawings

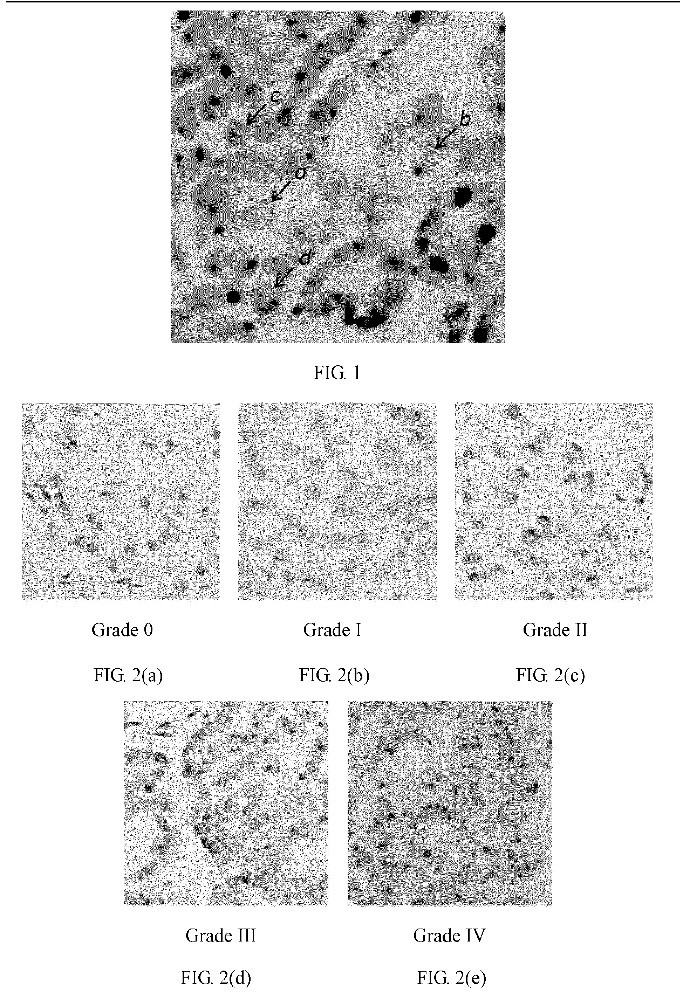

FIG 1 shows a pathological section containing thyroid cancer cells whose nuclei are stained with hematoxylin, with a indicating cells that, after being stained with hematoxylin, show no mark in the nucleus, meaning the imprinted gene is not expressed in the nucleus; b indicating cells that, after being stained with hematoxylin, show one red/brown mark in the nucleus, meaning the imprinted gene is present in the nucleus; c indicating cells that, after being stained with hematoxylin, show two red/brown marks in the nucleus, meaning the imprinted gene in the nucleus is affected by a loss of imprinting; and d indicating cells that, after being stained with hematoxylin, show more than two red/brown marks in the nucleus, meaning the imprinted gene in the nucleus has a copy number variation;

FIG 2(a) shows the expression states of ten genes in a pathological section of a Grade 0 thyroid tumor, FIG 2(b) shows the expression states of ten genes in a pathological section of Grade I thyroid cancer, FIG 2(c) shows the expression states of ten genes in a pathological section of Grade II thyroid cancer, FIG 2(d) shows the expression states of ten genes in a pathological section of Grade III thyroid cancer, and FIG 2(e) shows the expression states of ten genes in a pathological section of Grade IV thyroid cancer;

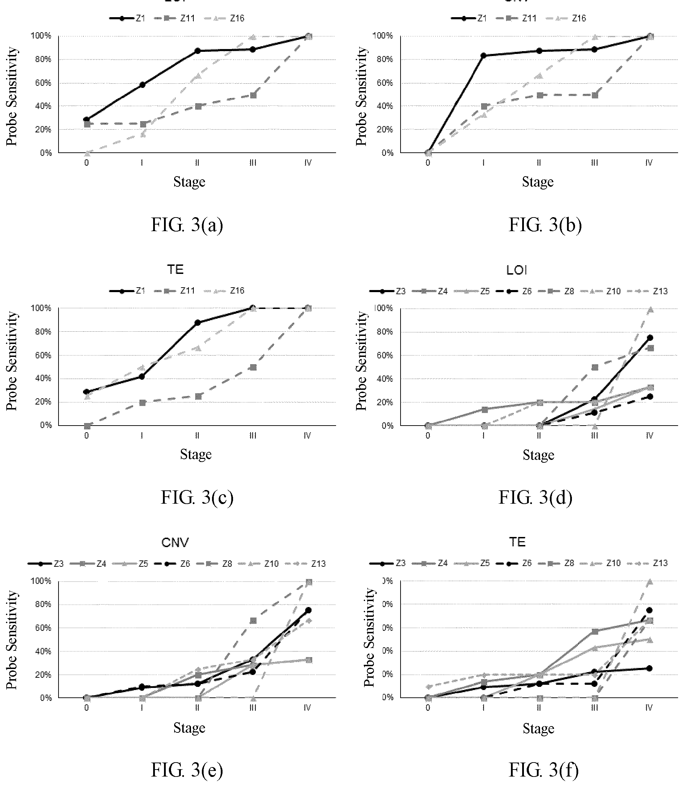

FIG 3(a) shows the intensities of the imprinted genes Z1, Z11, and Z16 as an indication of their respective expressed quantities corresponding to a loss of imprinting associated with thyroid cancer; FIG 3(b) shows the intensities of the imprinted genes Z1, Z11, and Z16 as an indication of their respective expressed quantities corresponding to a copy number variation associated with thyroid cancer; FIG 3(c) shows the intensities of the imprinted genes Z1, Z11, and Z16 as an indication of their respective total expressed quantities associated with thyroid cancer; FIG 3(d) shows the intensities of the imprinted genes Z3, Z4, Z5, Z6, Z8, Z10, and Z13 as an indication of their respective expressed quantities corresponding to a loss of imprinting associated with thyroid cancer; FIG 3(e) shows the intensities of the imprinted genes Z3, Z4, Z5, Z6, Z8, Z10, and Z13 as an indication of their respective expressed quantities corresponding to a copy number variation associated with thyroid cancer; and FIG. 3(f) shows the intensities of the imprinted genes Z3, Z4, Z5, Z6, Z8, Z10, and Z13 as an indication of their respective total expressed quantities associated with thyroid cancer, with LOI indicating the expressed quantities of imprinted genes with a loss of imprinting, CNV indicating the expressed quantities of imprinted genes with a copy number variation, and TE indicating the total expressed quantities of imprinted genes;

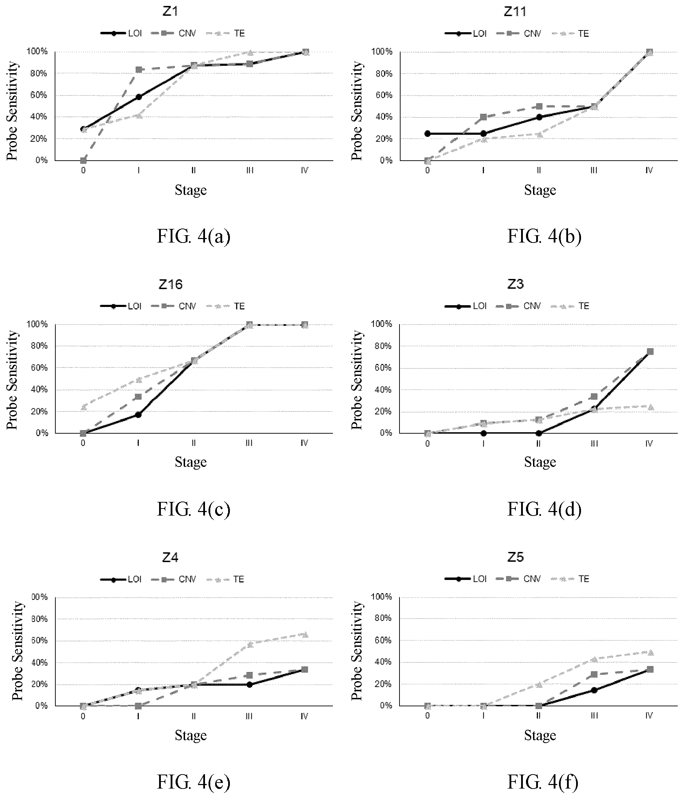

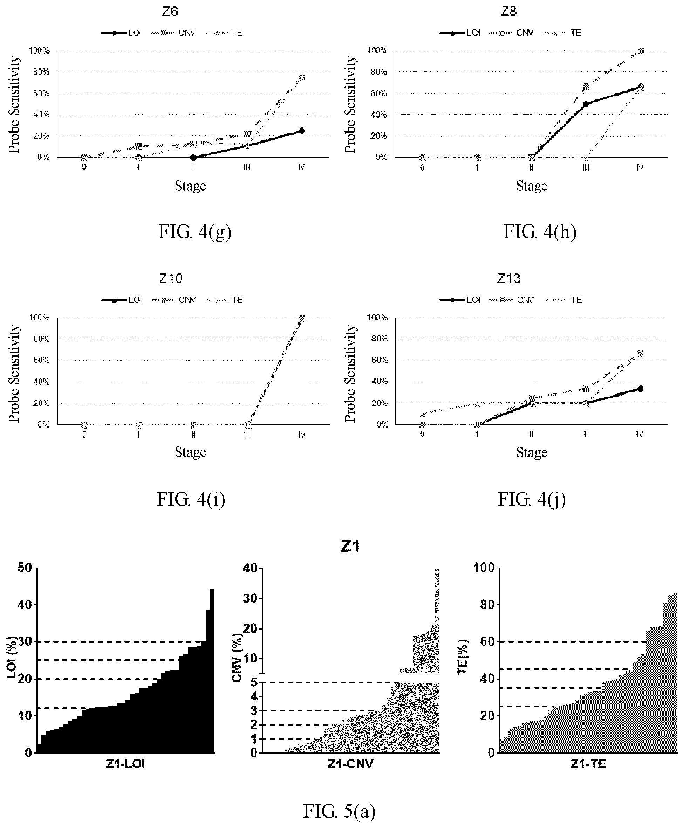

FIG 4(a) shows the intensities of the imprinted gene Z1 as an indication of its expressed quantities corresponding to a loss of imprinting or a copy number variation or of its total expressed quantities, FIG 4(b) shows the intensities of the imprinted gene Z11 as an indication of its expressed quantities corresponding to a loss of imprinting or a copy number variation or of its total expressed quantities, FIG. 4(c) shows the intensities of the imprinted gene Z16 as an indication of its expressed quantities corresponding to a loss of imprinting or a copy number variation or of its total expressed quantities, FIG 4(d) shows the intensities of the imprinted gene Z3 as an indication of its expressed quantities corresponding to a loss of imprinting or a copy number variation or of its total expressed quantities, FIG 4(e) shows the intensities of the imprinted gene Z4 as an indication of its expressed quantities corresponding to a loss of imprinting or a copy number variation or of its total expressed quantities, FIG 4(f) shows the intensities of the imprinted gene Z5 as an indication of its expressed quantities corresponding to a loss of imprinting or a copy number variation or of its total expressed quantities, FIG 4(g) shows the intensities of the imprinted gene Z6 as an indication of its expressed quantities corresponding to a loss of imprinting or a copy number variation or of its total expressed quantities, FIG 4(h) shows the intensities of the imprinted gene Z8 as an indication of its expressed quantities corresponding to a loss of imprinting or a copy number variation or of its total expressed quantities, FIG 4(i) shows the intensities of the imprinted gene Z10 as an indication of its expressed quantities corresponding to a loss of imprinting or a copy number variation or of its total expressed quantities, and FIG 4(j) shows the intensities of the imprinted gene Z13 as an indication of its expressed quantities corresponding to a loss of imprinting or a copy number variation or of its total expressed quantities, with LOI indicating the expressed quantities of an imprinted gene with a loss of imprinting, CNV indicating the expressed quantities of an imprinted gene with a copy number variation, and TE indicating the total expressed quantities of an imprinted gene; and

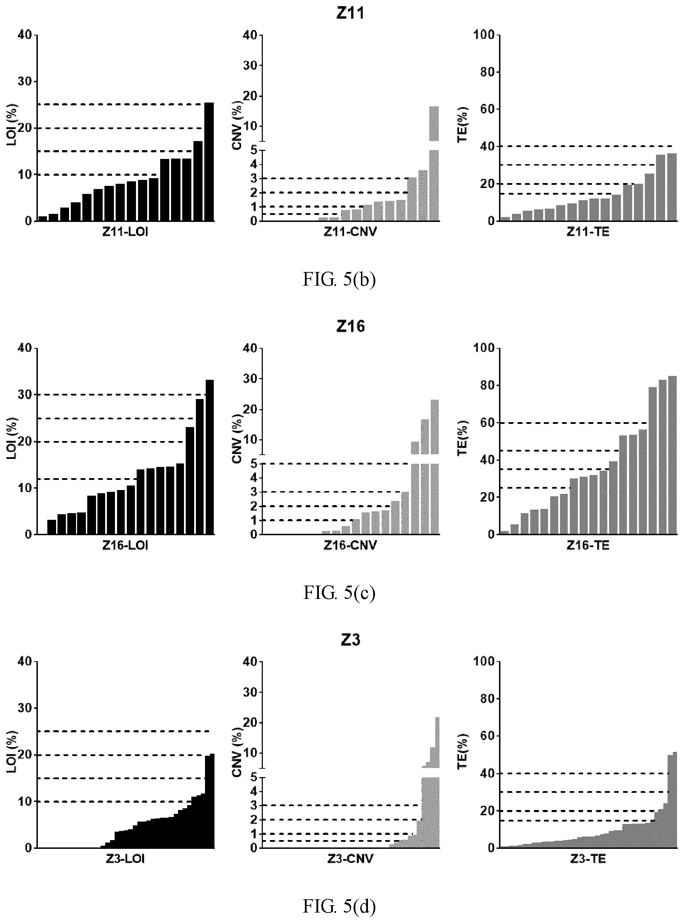

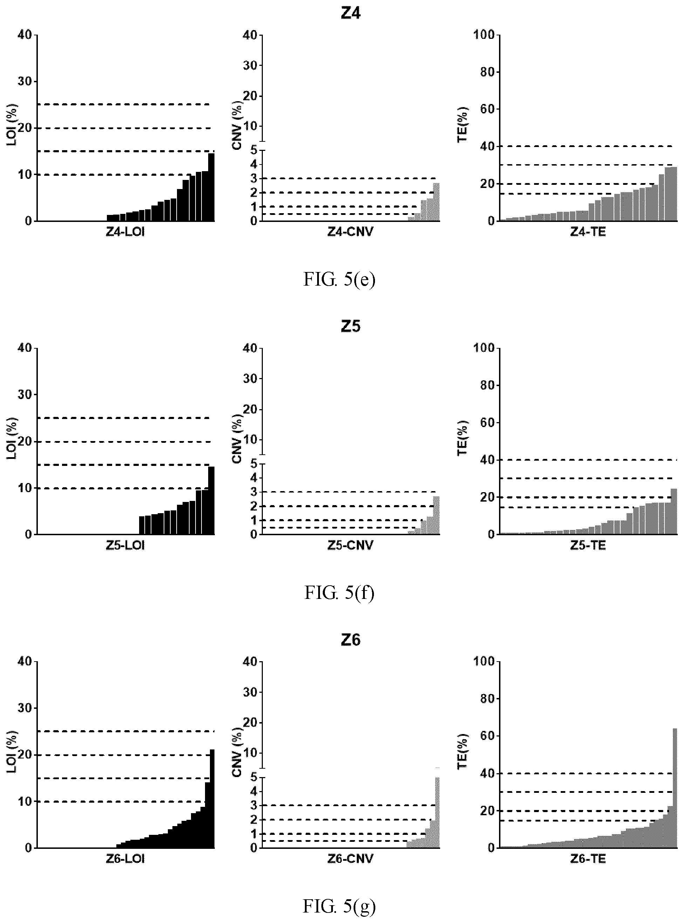

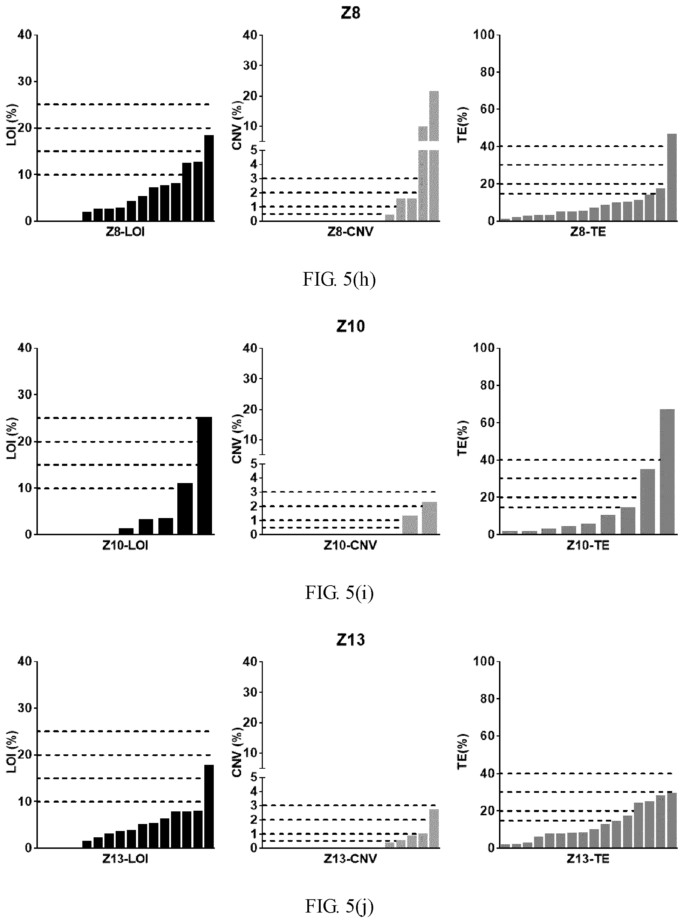

FIG 5(a) shows the distributions and grading criteria of the loss-of-imprinting-related and copy-number-variation-related expressed quantities of the imprinted gene Z1 in 41 pathological sections of thyroid cancer, FIG 5(b) shows the distributions and grading criteria of the loss-of-imprinting-related and copy-number-variation-related expressed quantities of the imprinted gene Z11 in 41 pathological sections of thyroid cancer, FIG 5(c) shows the distributions and grading criteria of the loss-of-imprinting-related and copy-number-variation-related expressed quantities of the imprinted gene Z16 in 41 pathological sections of thyroid cancer, FIG 5(d) shows the distributions and grading criteria of the loss-of-imprinting-related and copy-number-variation-related expressed quantities of the imprinted gene Z3 in 41 pathological sections of thyroid cancer, FIG 5(e) shows the distributions and grading criteria of the loss-of-imprinting-related and copy-number-variation-related expressed quantities of the imprinted gene Z4 in 41 pathological sections of thyroid cancer, FIG 5(f) shows the distributions and grading criteria of the loss-of-imprinting-related and copy-number-variation-related expressed quantities of the imprinted gene Z5 in 41 pathological sections of thyroid cancer, FIG 5(g) shows the distributions and grading criteria of the loss-of-imprinting-related and copy-number-variation-related expressed quantities of the imprinted gene Z6 in 41 pathological sections of thyroid cancer, FIG 5(h) shows the distributions and grading criteria of the loss-of-imprinting-related and copy-number-variation-related expressed quantities of the imprinted gene Z8 in 41 pathological sections of thyroid cancer, FIG 5(i) shows the distributions and grading criteria of the loss-of-imprinting-related and copy-number-variation-related expressed quantities of the imprinted gene Z10 in 41 pathological sections of thyroid cancer, and FIG 5(j) shows the distributions and grading criteria of the loss-of-imprinting-related and copy-number-variation-related expressed quantities of the imprinted gene Z13 in 41 pathological sections of thyroid cancer, with LOI indicating the expressed quantities of an imprinted gene with a loss of imprinting, CNV indicating the expressed quantities of an imprinted gene with a copy number variation, and TE indicating the total expressed quantities of an imprinted gene.

## Detailed Description of the Invention

The invention is defined by the appended claims.

To further explain the technical solution used by the present invention and its effects, certain embodiments are detailed below with reference to the accompanying drawings to expound the technical solution of the invention. The invention, however, is not limited to the embodiments described below.

Genomic imprinting is a gene regulation method in epigenetics and is characterized by methylating an allele from a specific parent such that only one allele of the corresponding gene is expressed while the other allele is in a silenced state. A gene regulated in this way is referred to as an imprinted gene. Loss of imprinting is an epigenetic change in which the silenced allele of an imprinted gene is demethylated and is thus activated and expressed. Numerous studies have shown that loss of imprinting exists widely in all kinds of cancers and takes place earlier than morphological changes in cells and tissue. Loss of imprinting, however, seldom occurs in healthy cells, which is in stark contrast to the case with cancer cells. Therefore, the methylated state of an imprinted gene can serve as a pathological marker and used in conjunction with specific molecular detection techniques to analyze cellular abnormality.

The present disclosure provides a detection model and device that enable intuitive observation of the expression of an imprinted gene with a loss of imprinting in a sample taken from a patient with a thyroid tumor. By labeling the imprinted gene in situ, changes in the imprinted gene can be objectively, intuitively, and precisely detected at an early stage. Not only that, a quantitative model is provided. Thus, the disclosure contributes greatly to the diagnosis of thyroid tumors.

The detection device disclosed herein can be used to determine the degree of benignity/malignancy of a patient's thyroid tumor through a needle biopsy sample before the patient is operated on, thus providing a basis for the surgical operation and precise treatment to be performed. This is a revolutionary breakthrough in the diagnosis of thyroid tumors on a cellular or molecular level.

The present disclosure makes it possible to precisely diagnose the type of a thyroid tumor, and by detecting a combination of imprinted genes, the degree of malignancy of the thyroid tumor can be graded in an unambiguous manner to enhance early definite diagnosis of thyroid cancer substantially. The disclosure is useful especially in early general surveys and cancer patients' postoperative follow-ups, in particular when cancer recurrence is suspected. The disclosure can shorten the time required for diagnosis and thus contributes greatly to saving human lives.

According to the present disclosure, the benignity or malignancy of a Hürthle cell tumor (HCT) can be accurately identified on a molecular level, thereby overcoming the current difficulty of making such a distinction in histomorphology or cytomorphology. The disclosure also solves the problem of discriminating between follicular thyroid adenomas (FTA) and follicular thyroid carcinomas (FTC), which is a problem encountered worldwide in morphological diagnosis.

The detection method disclosed herein is different from its immunohistochemical counterparts in that it can reduce false positives and other negative effects. Moreover, the discovery of targeting drugs or methods that work at the loss-of-imprinting-affected sites of thyroid-tumor-related imprinted genes to silence, delete, or rearrange those genes can be used to guide subsequent treatment and medication.

### Embodiment 1: imprinted gene analysis for thyroid cancer

The invention is defined by the appended claims.

The imprinted gene detection method used in this embodiment includes the following steps:

(1) Thyroid cancer tissue or cells were obtained and placed in 10% neutral buffered formalin in order to be fixed, lest the RNA degrade. After fixing for 24 hours and embedment in paraffin (FFPE), the embedded tissue or cells were cut into 10 $\mu$m-thick sections, which were loaded onto positively charged slides. The slides were then baked in a 40°C oven for at least 3 hours.

(2) The sample processing methods of RNAscope were used to dewax the sections, block the activity of the endogenous peroxidase in the samples, permeabilize the tissue or cells, and expose the RNA molecules.

(3) Probe design: Specific primers, or probes, were designed according to the sequences of the imprinted genes to be detected.

The probes were designed according to the to-be-detected imprinted genes Z1 (Gnas), Z3 (Peg10), Z4 (Igf2r), Z5 (Mest), Z6 (Plagl1), Z8 (Dcn), Z10 (Gatm), Z11 (Grb10), Z13 (Sgce), and Z16 (Snrpn/Snurf). More specifically, a sequence was selected from the intron of each of the aforesaid genes as the corresponding probe. The probes were designed by Advanced Cell Diagnostics.

(4) RNAscope reagent kits were used to perform in-situ hybridization between the probes in step (3) and the test samples.

(5) After staining with hematoxylin for signal amplification, the expression of the imprinted genes was analyzed via microscopic imaging.

The total expressed quantity of each imprinted gene, the expressed quantity of each imprinted gene with a loss of imprinting, and the expressed quantity of each imprinted gene with a copy number variation were calculated by the foregoing model using the following formulas:

$$\text{total expressed quantity of an imprinted gene} = (b+c+d)/(a+b+c+d) \times 100\%;$$

$$\text{expressed quantity of the imprinted gene being normal} = b/(b+c+d) \times 100\%;$$

$$\text{expressed quantity of the imprinted gene with a loss of imprinting (LOI)} = c/(b+c+d) \times 100\%;$$

and

$$\text{expressed quantity of the imprinted gene with a copy number variation (CNV)} = d/(b+c+d) \times 100\%;$$

where a, b, c, and d are shown in FIG 1, with a being the number of cells that, after being stained with hematoxylin, show no mark in the nucleus, meaning the imprinted gene is not expressed in the nucleus; b being the number of cells that, after being stained with hematoxylin, show one red/brown mark in the nucleus, meaning the imprinted gene is present in the nucleus; c being the number of cells that, after being stained with hematoxylin, show two red/brown marks in the nucleus, meaning the imprinted gene in the nucleus is affected by a loss of imprinting; and d being the number of cells that, after being stained with hematoxylin, show more than two red/brown marks in the nucleus, meaning the imprinted gene in the nucleus has a copy number variation.

As can be seen in the Grade 0 to Grade IV samples in FIG 2(a) to FIG 2(e), the proportions of cells with a loss of imprinting (i.e., cells with two signal points in the nucleus) and of cells with a copy number variation (i.e., cells with three or more signal points in the nucleus) gradually increased with the degree of malignancy.

**Embodiment 2: analysis of imprinted genes in needle biopsy samples of the thyroid**

The invention is defined by the appended claims.

The needle biopsy samples for use in this embodiment were suspicious lesion tissue extracted from the thyroid with a biopsy needle and were fixed with 10% neutral buffered formalin for at least 24 hours. The remaining steps of the detection method were the same as those in embodiment 1.

As can be seen in FIG 3(a) to FIG 3(f), each of the genes Z1, Z3, Z4, Z5, Z6, Z8, Z10, Z11, Z13, and Z16 had a different reaction sensitivity to thyroid cancer from another; in other words, the expression intensities and states of each gene with a loss of imprinting associated with thyroid cancer were different from those of another.

The sensitivity of each imprinted gene to thyroid cancer is shown in the corresponding plot in FIG 4(a) to FIG 4(j). Referring to FIG. 4(a), loss of imprinting and an increase of the total expressed quantity of the imprinted gene Z1 began to show in the potentially malignant stage, and the loss of imprinting and the total expressed quantity of the imprinted gene Z1 rose rapidly to very high levels in the early-stage thyroid cancer stage and remained at their respective levels in the intermediate-stage thyroid cancer stage through the advanced thyroid cancer stage; and copy number variation of the imprinted gene Z1 increased rapidly to a very high level in the potentially malignant stage and stayed at that level in the early-stage thyroid cancer stage through the advanced thyroid cancer stage. Referring to FIG 4(b), loss of imprinting, copy number variation, and the total expressed quantity of the imprinted gene Z11 were at relatively low levels in the potentially malignant stage, rose slowly in the early-stage thyroid cancer stage through the intermediate-stage thyroid cancer stage, and rocketed to very high levels in the advanced thyroid cancer stage. Referring to FIG 4(c), loss of imprinting, copy number variation, and the total expressed quantity of the imprinted gene Z16 increased rapidly in the potentially malignant stage through the intermediate-stage thyroid cancer stage and stayed at very high levels in the advanced thyroid cancer stage.

Referring to FIG 4(d), loss of imprinting and copy number variation of the imprinted gene Z3 did not increase much in the potentially malignant stage through the early-stage thyroid cancer stage, rose slowly in the intermediate-stage thyroid cancer stage, and went up rapidly to relatively high levels in the advanced thyroid cancer stage; and the total expressed quantity of the imprinted gene Z3 began to increase in the potentially malignant stage, rose slowly as the thyroid cancer developed, but was still very low in the advanced thyroid cancer stage. Referring to FIG 4(e), loss of imprinting and copy number variation of the imprinted gene Z4 increased slowly as the thyroid cancer developed but were still very low in the advanced thyroid cancer stage, and the total expressed quantity of the imprinted gene Z4 began to increase in the potentially malignant stage, did not increase much in the early-stage thyroid cancer stage, rose rapidly in the intermediate-stage thyroid cancer stage, and showed a relatively high level of sensitivity in the advanced thyroid cancer stage. Referring to FIG 4(f), loss of imprinting and copy number variation of the imprinted gene Z5 began to show in the intermediate-stage thyroid cancer stage but were still not high in the advanced thyroid cancer stage, and the total expressed quantity of the imprinted gene Z5 began to increase in the early-stage thyroid cancer stage, rose gradually as the thyroid cancer developed, but was still not high in the advanced thyroid cancer stage. Referring to FIG 4(g), loss of imprinting of the imprinted gene Z6 began to show in the intermediate-stage thyroid cancer stage, rose to a certain degree in the advanced thyroid cancer stage, but was still not high; copy number variation of the imprinted gene Z6 began to show in the potential thyroid cancer stage, remained stable in the early-stage thyroid cancer stage through the intermediate-stage thyroid cancer stage, and rose rapidly to a relatively high level in the advanced thyroid cancer stage; and the total expressed quantity of the imprinted gene Z6 began to increase in the early-stage thyroid cancer stage, remained stable in the intermediate-stage thyroid cancer stage, and rose rapidly to a relatively high level in the advanced thyroid cancer stage. Referring to FIG 4(h), loss of imprinting of the imprinted gene Z8 increased rapidly in the intermediate-stage thyroid cancer stage and kept rising to a relatively high level in the advanced thyroid cancer stage, copy number variation of the imprinted gene Z8 increased rapidly in the intermediate-stage thyroid cancer stage and kept rising to a very high level in the advanced thyroid cancer stage, and the total expressed quantity of the imprinted gene Z8 began to increase in the advanced thyroid cancer stage and reached a relatively high level. Referring to FIG 4(i), loss of imprinting, copy number variation, and an increase of the total expressed quantity of the imprinted gene Z10 began to show in the advanced thyroid cancer stage, and the loss of imprinting, the copy number variation, and the total expressed quantity of the imprinted gene Z10 reached very high levels. Referring to FIG 4(j), loss of imprinting of the imprinted gene Z13 began to show in the early-stage thyroid cancer stage and rose slowly in the intermediate-stage thyroid cancer stage through the advanced thyroid cancer stage; copy number variation of the imprinted gene Z13 began to show in the early-stage thyroid cancer stage, rose slowly in the intermediate-stage thyroid cancer stage, and went up rapidly to a relatively high level in the advanced thyroid cancer stage; and the total expressed quantity of the imprinted gene Z13 began to increase in the potentially malignant stage, did not increase much in the early-stage thyroid cancer stage through the intermediate-stage thyroid cancer stage, and rose rapidly to a relatively high level in the advanced thyroid cancer stage.

**Embodiment 3: analysis of imprinted genes in 41 thyroid tumor samples**

The invention is defined by the appended claims.

41 tissue samples, including cell samples obtained through needle biopsies, were obtained from thyroid cancer patients. The detection method used was the same as that in embodiment 1.

FIG 5(a) to FIG 5(j) show the distributions of the expressed quantities (arranged in order of increasing percentage) corresponding respectively to a loss of imprinting and a copy number variation as detected by the ten probes used in the thyroid tumor tissue samples. Based on the trends of distribution corresponding respectively to the different probes, we calculated the dashed-line grading criteria in each plot so as to classify the expressed quantities corresponding respectively to a loss of imprinting and a copy number variation as detected by each probe into five grades in order of increasing magnitude.

More specifically, the grading was carried out as follows:

Regarding the imprinted gene Z1, referring to FIG 5(a), Grade 0 was given when any one or at least two of the following conditions were met: the expressed quantity of the imprinted gene with a loss of imprinting is less than 12%, the expressed quantity of the imprinted gene with a copy number variation is less than 1%, and the total expressed quantity of the imprinted gene is less than 25%; Grade I was given when any one or at least two of the following conditions were met: the expressed quantity of the imprinted gene with a loss of imprinting is 12%-20%, the expressed quantity of the imprinted gene with a copy number variation is 1%-2%, and the total expressed quantity of the imprinted gene is 25%-35%; Grade II was given when any one or at least two of the following conditions were met: the expressed quantity of the imprinted gene with a loss of imprinting is 20%-25%, the expressed quantity of the imprinted gene with a copy number variation is 2%-3%, and the total expressed quantity of the imprinted gene is 35%-45%; Grade III was given when any one or at least two of the following conditions were met: the expressed quantity of the imprinted gene with a loss of imprinting is 25%-30%, the expressed quantity of the imprinted gene with a copy number variation is 3%-5%, and the total expressed quantity of the imprinted gene is 45%-60%; and Grade IV was given when any one or at least two of the following conditions were met: the expressed quantity of the imprinted gene with a loss of imprinting is greater than 30%, the expressed quantity of the imprinted gene with a copy number variation is greater than 5%, and the total expressed quantity of the imprinted gene is greater than 60%.

Regarding the imprinted gene Z11, referring to FIG 5(b), Grade 0 was given when any one or at least two of the following conditions were met: the expressed quantity of the imprinted gene with a loss of imprinting is less than 10%, the expressed quantity of the imprinted gene with a copy number variation is less than 0.5%, and the total expressed quantity of the imprinted gene is less than 15%; Grade I was given when any one or at least two of the following conditions were met: the expressed quantity of the imprinted gene with a loss of imprinting is 10%-15%, the expressed quantity of the imprinted gene with a copy number variation is 0.5%-1%, and the total expressed quantity of the imprinted gene is 15%-20%; Grade II was given when any one or at least two of the following conditions were met: the expressed quantity of the imprinted gene with a loss of imprinting is 15%-20%, the expressed quantity of the imprinted gene with a copy number variation is 1%-2%, and the total expressed quantity of the imprinted gene is 20%-30%; Grade III was given when any one or at least two of the following conditions were met: the expressed quantity of the imprinted gene with a loss of imprinting is 20%-25%, the expressed quantity of the imprinted gene with a copy number variation is 2%-3%, and the total expressed quantity of the imprinted gene is 30%-40%; and Grade IV was given when any one or at least two of the following conditions were met: the expressed quantity of the imprinted gene with a loss of imprinting is greater than 25%, the expressed quantity of the imprinted gene with a copy number variation is greater than 3%, and the total expressed quantity of the imprinted gene is greater than 40%.

Regarding the imprinted gene Z16, referring to FIG 5(c), Grade 0 was given when any one or at least two of the following conditions were met: the expressed quantity of the imprinted gene with a loss of imprinting is less than 12%, the expressed quantity of the imprinted gene with a copy number variation is less than 1%, and the total expressed quantity of the imprinted gene is less than 25%; Grade I was given when any one or at least two of the following conditions were met: the expressed quantity of the imprinted gene with a loss of imprinting is 12%-20%, the expressed quantity of the imprinted gene with a copy number variation is 1%-2%, and the total expressed quantity of the imprinted gene is 25%-35%; Grade II was given when any one or at least two of the following conditions were met: the expressed quantity of the imprinted gene with a loss of imprinting is 20%-25%, the expressed quantity of the imprinted gene with a copy number variation is 2%-3%, and the total expressed quantity of the imprinted gene is 35%-45%; Grade III was given when any one or at least two of the following conditions were met: the expressed quantity of the imprinted gene with a loss of imprinting is 25%-30%, the expressed quantity of the imprinted gene with a copy number variation is 3%-5%, and the total expressed quantity of the imprinted gene is 45%-60%; and Grade IV was given when any one or at least two of the following conditions were met: the expressed quantity of the imprinted gene with a loss of imprinting is greater than 30%, the expressed quantity of the imprinted gene with a copy number variation is greater than 5%, and the total expressed quantity of the imprinted gene is greater than 60%.

Regarding the imprinted gene Z3, referring to FIG 5(d), Grade 0 was given when any one or at least two of the following conditions were met: the expressed quantity of the imprinted gene with a loss of imprinting is less than 10%, the expressed quantity of the imprinted gene with a copy number variation is less than 0.5%, and the total expressed quantity of the imprinted gene is less than 15%; Grade I was given when any one or at least two of the following conditions were met: the expressed quantity of the imprinted gene with a loss of imprinting is 10%-15%, the expressed quantity of the imprinted gene with a copy number variation is 0.5%-1%, and the total expressed quantity of the imprinted gene is 15%-20%; Grade II was given when any one or at least two of the following conditions were met: the expressed quantity of the imprinted gene with a loss of imprinting is 15%-20%, the expressed quantity of the imprinted gene with a copy number variation is 1%-2%, and the total expressed quantity of the imprinted gene is 20%-30%; Grade III was given when any one or at least two of the following conditions were met: the expressed quantity of the imprinted gene with a loss of imprinting is 20%-25%, the expressed quantity of the imprinted gene with a copy number variation is 2%-3%, and the total expressed quantity of the imprinted gene is 30%-40%; and Grade IV was given when any one or at least two of the following conditions were met: the expressed quantity of the imprinted gene with a loss of imprinting is greater than 25%, the expressed quantity of the imprinted gene with a copy number variation is greater than 3%, and the total expressed quantity of the imprinted gene is greater than 40%.

Regarding the imprinted gene Z4, referring to FIG 5(e), Grade 0 was given when any one or at least two of the following conditions were met: the expressed quantity of the imprinted gene with a loss of imprinting is less than 10%, the expressed quantity of the imprinted gene with a copy number variation is less than 0.5%, and the total expressed quantity of the imprinted gene is less than 15%; Grade I was given when any one or at least two of the following conditions were met: the expressed quantity of the imprinted gene with a loss of imprinting is 10%-15%, the expressed quantity of the imprinted gene with a copy number variation is 0.5%-1%, and the total expressed quantity of the imprinted gene is 15%-20%; Grade II was given when any one or at least two of the following conditions were met: the expressed quantity of the imprinted gene with a loss of imprinting is 15%-20%, the expressed quantity of the imprinted gene with a copy number variation is 1%-2%, and the total expressed quantity of the imprinted gene is 20%-30%; Grade III was given when any one or at least two of the following conditions were met: the expressed quantity of the imprinted gene with a loss of imprinting is 20%-25%, the expressed quantity of the imprinted gene with a copy number variation is 2%-3%, and the total expressed quantity of the imprinted gene is 30%-40%; and Grade IV was given when any one or at least two of the following conditions were met: the expressed quantity of the imprinted gene with a loss of imprinting is greater than 25%, the expressed quantity of the imprinted gene with a copy number variation is greater than 3%, and the total expressed quantity of the imprinted gene is greater than 40%.

Regarding the imprinted gene Z5, referring to FIG. 5(f), Grade 0 was given when any one or at least two of the following conditions were met: the expressed quantity of the imprinted gene with a loss of imprinting is less than 10%, the expressed quantity of the imprinted gene with a copy number variation is less than 0.5%, and the total expressed quantity of the imprinted gene is less than 15%; Grade I was given when any one or at least two of the following conditions were met: the expressed quantity of the imprinted gene with a loss of imprinting is 10%-15%, the expressed quantity of the imprinted gene with a copy number variation is 0.5%-1%, and the total expressed quantity of the imprinted gene is 15%-20%; Grade II was given when any one or at least two of the following conditions were met: the expressed quantity of the imprinted gene with a loss of imprinting is 15%-20%, the expressed quantity of the imprinted gene with a copy number variation is 1%-2%, and the total expressed quantity of the imprinted gene is 20%-30%; Grade III was given when any one or at least two of the following conditions were met: the expressed quantity of the imprinted gene with a loss of imprinting is 20%-25%, the expressed quantity of the imprinted gene with a copy number variation is 2%-3%, and the total expressed quantity of the imprinted gene is 30%-40%; and Grade IV was given when any one or at least two of the following conditions were met: the expressed quantity of the imprinted gene with a loss of imprinting is greater than 25%, the expressed quantity of the imprinted gene with a copy number variation is greater than 3%, and the total expressed quantity of the imprinted gene is greater than 40%.

Regarding the imprinted gene Z6, referring to FIG 5(g), Grade 0 was given when any one or at least two of the following conditions were met: the expressed quantity of the imprinted gene with a loss of imprinting is less than 10%, the expressed quantity of the imprinted gene with a copy number variation is less than 0.5%, and the total expressed quantity of the imprinted gene is less than 15%; Grade I was given when any one or at least two of the following conditions were met: the expressed quantity of the imprinted gene with a loss of imprinting is 10%-15%, the expressed quantity of the imprinted gene with a copy number variation is 0.5%-1%, and the total expressed quantity of the imprinted gene is 15%-20%; Grade II was given when any one or at least two of the following conditions were met: the expressed quantity of the imprinted gene with a loss of imprinting is 15%-20%, the expressed quantity of the imprinted gene with a copy number variation is 1%-2%, and the total expressed quantity of the imprinted gene is 20%-30%; Grade III was given when any one or at least two of the following conditions were met: the expressed quantity of the imprinted gene with a loss of imprinting is 20%-25%, the expressed quantity of the imprinted gene with a copy number variation is 2%-3%, and the total expressed quantity of the imprinted gene is 30%-40%; and Grade IV was given when any one or at least two of the following conditions were met: the expressed quantity of the imprinted gene with a loss of imprinting is greater than 25%, the expressed quantity of the imprinted gene with a copy number variation is greater than 3%, and the total expressed quantity of the imprinted gene is greater than 40%.

Regarding the imprinted gene Z8, referring to FIG 5(h), Grade 0 was given when any one or at least two of the following conditions were met: the expressed quantity of the imprinted gene with a loss of imprinting is less than 10%, the expressed quantity of the imprinted gene with a copy number variation is less than 0.5%, and the total expressed quantity of the imprinted gene is less than 15%; Grade I was given when any one or at least two of the following conditions were met: the expressed quantity of the imprinted gene with a loss of imprinting is 10%-15%, the expressed quantity of the imprinted gene with a copy number variation is 0.5%-1%, and the total expressed quantity of the imprinted gene is 15%-20%; Grade II was given when any one or at least two of the following conditions were met: the expressed quantity of the imprinted gene with a loss of imprinting is 15%-20%, the expressed quantity of the imprinted gene with a copy number variation is 1%-2%, and the total expressed quantity of the imprinted gene is 20%-30%; Grade III was given when any one or at least two of the following conditions were met: the expressed quantity of the imprinted gene with a loss of imprinting is 20%-25%, the expressed quantity of the imprinted gene with a copy number variation is 2%-3%, and the total expressed quantity of the imprinted gene is 30%-40%; and Grade IV was given when any one or at least two of the following conditions were met: the expressed quantity of the imprinted gene with a loss of imprinting is greater than 25%, the expressed quantity of the imprinted gene with a copy number variation is greater than 3%, and the total expressed quantity of the imprinted gene is greater than 40%.

Regarding the imprinted gene Z10, referring to FIG 5(i), Grade 0 was given when any one or at least two of the following conditions were met: the expressed quantity of the imprinted gene with a loss of imprinting is less than 10%, the expressed quantity of the imprinted gene with a copy number variation is less than 0.5%, and the total expressed quantity of the imprinted gene is less than 15%; Grade I was given when any one or at least two of the following conditions were met: the expressed quantity of the imprinted gene with a loss of imprinting is 10%-15%, the expressed quantity of the imprinted gene with a copy number variation is 0.5%-1%, and the total expressed quantity of the imprinted gene is 15%-20%; Grade II was given when any one or at least two of the following conditions were met: the expressed quantity of the imprinted gene with a loss of imprinting is 15%-20%, the expressed quantity of the imprinted gene with a copy number variation is 1%-2%, and the total expressed quantity of the imprinted gene is 20%-30%; Grade III was given when any one or at least two of the following conditions were met: the expressed quantity of the imprinted gene with a loss of imprinting is 20%-25%, the expressed quantity of the imprinted gene with a copy number variation is 2%-3%, and the total expressed quantity of the imprinted gene is 30%-40%; and Grade IV was given when any one or at least two of the following conditions were met: the expressed quantity of the imprinted gene with a loss of imprinting is greater than 25%, the expressed quantity of the imprinted gene with a copy number variation is greater than 3%, and the total expressed quantity of the imprinted gene is greater than 40%.

Regarding the imprinted gene Z13, referring to FIG 5(j), Grade 0 was given when any one or at least two of the following conditions were met: the expressed quantity of the imprinted gene with a loss of imprinting is less than 10%, the expressed quantity of the imprinted gene with a copy number variation is less than 0.5%, and the total expressed quantity of the imprinted gene is less than 15%; Grade I was given when any one or at least two of the following conditions were met: the expressed quantity of the imprinted gene with a loss of imprinting is 10%-15%, the expressed quantity of the imprinted gene with a copy number variation is 0.5%-1%, and the total expressed quantity of the imprinted gene is 15%-20%; Grade II was given when any one or at least two of the following conditions were met: the expressed quantity of the imprinted gene with a loss of imprinting is 15%-20%, the expressed quantity of the imprinted gene with a copy number variation is 1%-2%, and the total expressed quantity of the imprinted gene is 20%-30%; Grade III was given when any one or at least two of the following conditions were met: the expressed quantity of the imprinted gene with a loss of imprinting is 20%-25%, the expressed quantity of the imprinted gene with a copy number variation is 2%-3%, and the total expressed quantity of the imprinted gene is 30%-40%; and Grade IV was given when any one or at least two of the following conditions were met: the expressed quantity of the imprinted gene with a loss of imprinting is greater than 25%, the expressed quantity of the imprinted gene with a copy number variation is greater than 3%, and the total expressed quantity of the imprinted gene is greater than 40%.

An integrated analysis of the 41 thyroid tumor samples led to the following classification:

The degree of benignity/malignancy of a thyroid tumor can be classified as one of the following: benign tumor, potential thyroid cancer, early-stage thyroid cancer, intermediate-stage thyroid cancer, and advanced thyroid cancer, as detailed below:

the degree of benignity/malignancy of a thyroid tumor is determined as benign tumor when the expressed quantities of each of the imprinted genes Z1, Z3, Z4, Z5, Z6, Z8, Z10, Z11, Z13, and Z16 with, respectively, a loss of imprinting and a copy number variation are both graded as Grade 0; or when the expressed quantity of not more than one of the imprinted genes Z1, Z3, Z4, Z5, Z6, Z8, Z10, Z11, Z13, and Z16 with a loss of imprinting is graded as Grade I, and the expressed quantity of not more than one of the imprinted genes Z1, Z3, Z4, Z5, Z6, Z8, Z10, Z11, Z13, and Z16 with a copy number variation is graded as Grade I;
the degree of benignity/malignancy of the thyroid tumor is determined as potential thyroid cancer when the expressed quantities of at least two of the imprinted genes Z1, Z3, Z4, Z5, Z6, Z8, Z10, Z11, Z13, and Z16 with a loss of imprinting are graded as Grade I; or when the expressed quantities of at least two of the imprinted genes Z1, Z3, Z4, Z5, Z6, Z8, Z10, Z11, Z13, and Z16 with a copy number variation are graded as Grade I; or when the expressed quantity of not more than one of the imprinted genes Z1, Z3, Z4, Z5, Z6, Z8, Z10, Z11, Z13, and Z16 with a loss of imprinting is graded as Grade II, and the expressed quantity of not more than one of the imprinted genes Z1, Z3, Z4, Z5, Z6, Z8, Z10, Z11, Z13, and Z16 with a copy number variation is graded as Grade II;
the degree of benignity/malignancy of the thyroid tumor is determined as early-stage thyroid cancer when the expressed quantities of at least two of the imprinted genes Z1, Z3, Z4, Z5, Z6, Z8, Z10, Z11, Z13, and Z16 with a loss of imprinting are graded as Grade II; or when the expressed quantities of at least two of the imprinted genes Z1, Z3, Z4, Z5, Z6, Z8, Z10, Z11, Z13, and Z16 with a copy number variation are graded as Grade II; or when the expressed quantity of not more than one of the imprinted genes Z1, Z3, Z4, Z5, Z6, Z8, Z10, Z11, Z13, and Z16 with a loss of imprinting is graded as Grade III, and the expressed quantity of not more than one of the imprinted genes Z1, Z3, Z4, Z5, Z6, Z8, Z10, Z11, Z13, and Z16 with a copy number variation is graded as Grade III;
the degree of benignity/malignancy of the thyroid tumor is determined as intermediate-stage thyroid cancer when the expressed quantities of at least two of the imprinted genes Z1, Z3, Z4, Z5, Z6, Z8, Z10, Z11, Z13, and Z16 with a loss of imprinting are graded as Grade III; or when the expressed quantities of at least two of the imprinted genes Z1, Z3, Z4, Z5, Z6, Z8, Z10, Z11, Z13, and Z16 with a copy number variation are graded as Grade III; or when the expressed quantity of not more than one of the imprinted genes Z1, Z3, Z4, Z5, Z6, Z8, Z10, Z11, Z13, and Z16 with a loss of imprinting is graded as Grade IV, and the expressed quantity of not more than one of the imprinted genes Z1, Z3, Z4, Z5, Z6, Z8, Z10, Z11, Z13, and Z16 with a copy number variation is graded as Grade IV; and
the degree of benignity/malignancy of the thyroid tumor is determined as advanced thyroid cancer when the expressed quantities of at least two of the imprinted genes Z1, Z3, Z4, Z5, Z6, Z8, Z10, Z11, Z13, and Z16 with a loss of imprinting are graded as Grade IV; or when the expressed quantities of at least two of the imprinted genes Z1, Z3, Z4, Z5, Z6, Z8, Z10, Z11, Z13, and Z16 with a copy number variation are graded as Grade IV

In summary of the above, the detection model and system of the present invention enable intuitive observation of the expression of an imprinted gene with a loss of imprinting in a sample taken from a patient with a thyroid tumor. By labeling the imprinted gene in situ, changes in the imprinted gene can be objectively, intuitively, and precisely detected at an early stage. Not only that, a quantitative model is provided. Thus, the invention contributes greatly to the diagnosis of thyroid tumors.

**Claims**

1. An imprinted gene grading model applicable to a thyroid tumor, the model being configured to calculate changes in the following expressed quantities of an imprinted gene in the thyroid tumor and grade an expression state of the imprinted gene accordingly: a total expressed quantity of the imprinted gene, an expressed quantity of the imprinted gene with a loss of imprinting, and an expressed quantity of the imprinted gene with a copy number variation;

wherein the imprinted gene is any one, or a combination of at least two, of Z1, Z11, and Z16, with the imprinted gene Z1 being Gnas, the imprinted gene Z11 being Grb10, and the imprinted gene Z16 being Snrpn/Snurf;
wherein the total expressed quantity of the imprinted gene, the expressed quantity of the imprinted gene with a loss of imprinting, and the expressed quantity of the imprinted gene with a copy number variation are calculated by the following formulas:

the total expressed quantity of the imprinted gene = $(b+c+d)/(a+b+c+d) \times 100\%$;

an expressed quantity of the imprinted gene being normal = $b/(b+c+d) \times 100\%$;

the expressed quantity of the imprinted gene with a loss of imprinting (LOI) = $c/(b+c+d) \times 100\%$;

and

the expressed quantity of the imprinted gene with a copy number variation (CNV) = $d/(b+c+d) \times 100\%$;

where a is the number of cells that, after being stained with hematoxylin, show no mark in the nucleus of each said cell, meaning the imprinted gene is not expressed in the nucleus of each said cell; b is the number of cells that, after being stained with hematoxylin, show one red/brown mark in the nucleus of each said cell, meaning the imprinted gene is present in the nucleus of each said cell; c is the number of cells that, after being stained with hematoxylin, show two red/brown marks in the nucleus of each said cell, meaning the imprinted gene in the nucleus of each said cell is affected by a loss of imprinting; and d is the number of cells that, after being stained with hematoxylin, show more than two red/brown marks in the nucleus of each said cell, meaning the imprinted gene in the nucleus of each said cell has a copy number variation; and

wherein the expressed quantity of the imprinted gene with a loss of imprinting, the expressed quantity of the imprinted gene with a copy number variation, and the total expressed quantity of the imprinted gene are each graded as one of five different grades;

the expressed quantity of each of the imprinted genes Z1 and Z16 with a loss of imprinting, the expressed quantity of each of the imprinted genes Z1 and Z16 with a copy number variation, and the total expressed quantity of each of the imprinted genes Z1 and Z16 are graded into the following five grades:

Grade 0: applicable when any one or at least two of the following conditions are met: the expressed quantity of one of the imprinted genes Z1 and Z16 with a loss of imprinting is less than 12%, the expressed quantity of one of the imprinted genes Z1 and Z16 with a copy number variation is less than 1%, and the total expressed quantity of one of the imprinted genes Z1 and Z16 is less than 25%;

Grade I: applicable when any one or at least two of the following conditions are met: the expressed quantity of one of the imprinted genes Z1 and Z16 with a loss of imprinting is 12%-20%, the expressed quantity of one of the imprinted genes Z1 and Z16 with a copy number variation is 1%-2%, and the total expressed quantity of one of the imprinted genes Z1 and Z16 is 25%-35%;

Grade II: applicable when any one or at least two of the following conditions are met: the expressed quantity of one of the imprinted genes Z1 and Z16 with a loss of imprinting is 20%-25%, the expressed quantity of one of the imprinted genes Z1 and Z16 with a copy number variation is 2%-3%, and the total expressed quantity of one of the imprinted genes Z1 and Z16 is 35%-45%;

Grade III: applicable when any one or at least two of the following conditions are met: the expressed quantity of one of the imprinted genes Z1 and Z16 with a loss of imprinting is 25%-30%, the expressed quantity of one of the imprinted genes Z1 and Z16 with a copy number variation is 3%-5%, and the total expressed quantity of one of the imprinted genes Z1 and Z16 is 45%-60%; and

Grade IV: applicable when any one or at least two of the following conditions are met: the expressed quantity of one of the imprinted genes Z1 and Z16 with a loss of imprinting is greater than 30%, the expressed quantity of one of the imprinted genes Z1 and Z16 with a copy number variation is greater than 5%, and the total expressed quantity of one of the imprinted genes Z1 and Z16 is greater than 60%; and

the expressed quantity of the imprinted gene Z11 with a loss of imprinting, the expressed quantity of the imprinted gene Z11 with a copy number variation, and the total expressed quantity of the imprinted gene Z11 are graded into the following five grades:

Grade 0: applicable when any one or at least two of the following conditions are met: the expressed quantity of the imprinted gene Z11 with a loss of imprinting is less than 10%, the expressed quantity of the imprinted gene Z11 with a copy number variation is less than 0.5%, and the total expressed quantity of the imprinted gene Z11 is less than 15%;

Grade I: applicable when any one or at least two of the following conditions are met: the expressed quantity of the imprinted gene Z11 with a loss of imprinting is 10%-15%, the expressed quantity of the imprinted gene Z11 with a copy number variation is 0.5%-1%, and the total expressed quantity of the imprinted gene Z11 is 15%-20%;

Grade II: applicable when any one or at least two of the following conditions are met: the expressed quantity of the imprinted gene Z11 with a loss of imprinting is 15%-20%, the expressed quantity of the imprinted gene Z11 with a copy number variation is 1%-2%, and the total expressed quantity of the imprinted gene Z11 is 20%-30%;

Grade III: applicable when any one or at least two of the following conditions are met: the expressed quantity of the imprinted gene Z11 with a loss of imprinting is 20%-25%, the expressed quantity of the imprinted gene Z11 with a copy number variation is 2%-3%, and the total expressed quantity of the imprinted gene Z11 is 30%-40%; and

Grade IV: applicable when any one or at least two of the following conditions are met: the expressed quantity of the imprinted gene Z11 with a loss of imprinting is greater than 25%, the expressed quantity of the imprinted gene Z11 with a copy number variation is greater than 3%, and the total expressed quantity of the imprinted gene Z11 is greater than 40%.

2. The model of claim 1, wherein the model calculates the expressed quantities of any one of the imprinted genes Z1, Z11, and Z16, preferably the imprinted gene Z1 or Z16, and more preferably the imprinted gene Z1.

3. The model of claim 1 or 2, wherein the model calculates the expressed quantities of a combination of any two of the imprinted genes Z1, Z11, and Z16, preferably a combination of the imprinted genes Z1 and Z16 or a combination of the imprinted genes Z1 and Z11.

4. The model of any of claims 1-3, wherein the imprinted gene further comprises any one, or a combination of at least two, of Z3, Z4, Z5, Z6, Z8, Z10, and Z13, with the imprinted gene Z3 being Peg10, the imprinted gene Z4 being Igf2r, the imprinted gene Z5 being Mest, the imprinted gene Z6 being Plagl1, the imprinted gene Z8 being Dcn, the imprinted gene Z10 being Gatm, and the imprinted gene Z13 being Sgce;
the expressed quantity of each of the imprinted genes Z3, Z4, Z5, Z6, Z8, Z10, and Z13 with a loss of imprinting, the expressed quantity of each of the imprinted genes Z3, Z4, Z5, Z6, Z8, Z10, and Z13 with a copy number variation, and the total expressed quantity of each of the imprinted genes Z3, Z4, Z5, Z6, Z8, Z10, and Z13 are graded into the following five grades:

Grade 0: applicable when any one or at least two of the following conditions are met: the expressed quantity of one of the imprinted genes Z3, Z4, Z5, Z6, Z8, Z10, and Z13 with a loss of imprinting is less than 10%, the expressed quantity of one of the imprinted genes Z3, Z4, Z5, Z6, Z8, Z10, and Z13 with a copy number variation is less than 0.5%, and the total expressed quantity of one of the imprinted genes Z3, Z4, Z5, Z6, Z8, Z10, and Z13 is less than 15%;
Grade I: applicable when any one or at least two of the following conditions are met: the expressed quantity of one of the imprinted genes Z3, Z4, Z5, Z6, Z8, Z10, and Z13 with a loss of imprinting is 10%-15%, the expressed quantity of one of the imprinted genes Z3, Z4, Z5, Z6, Z8, Z10, and Z13 with a copy number variation is 0.5%-1%, and the total expressed quantity of one of the imprinted genes Z3, Z4, Z5, Z6, Z8, Z10, and Z13 is 15%-20%;
Grade II: applicable when any one or at least two of the following conditions are met: the expressed quantity of one of the imprinted genes Z3, Z4, Z5, Z6, Z8, Z10, and Z13 with a loss of imprinting is 15%-20%, the expressed quantity of one of the imprinted genes Z3, Z4, Z5, Z6, Z8, Z10, and Z13 with a copy number variation is 1%-2%, and the total expressed quantity of one of the imprinted genes Z3, Z4, Z5, Z6, Z8, Z10, and Z13 is 20%-30%;
Grade III: applicable when any one or at least two of the following conditions are met: the expressed quantity of one of the imprinted genes Z3, Z4, Z5, Z6, Z8, Z10, and Z13 with a loss of imprinting is 20%-25%, the expressed quantity of one of the imprinted genes Z3, Z4, Z5, Z6, Z8, Z10, and Z13 with a copy number variation is 2%-3%, and the total expressed quantity of one of the imprinted genes Z3, Z4, Z5, Z6, Z8, Z10, and Z13 is 30%-40%; and
Grade IV: applicable when any one or at least two of the following conditions are met: the expressed quantity of one of the imprinted genes Z3, Z4, Z5, Z6, Z8, Z10, and Z13 with a loss of imprinting is greater than 25%, the expressed quantity of one of the imprinted genes Z3, Z4, Z5, Z6, Z8, Z10, and Z13 with a copy number variation is greater than 3%, and the total expressed quantity of one of the imprinted genes Z3, Z4, Z5, Z6, Z8, Z10, and Z13 is greater than 40%.

5. The model of any of claims 1-4 , wherein the model calculates the expressed quantities of a combination of the ten imprinted genes Z1, Z3, Z4, Z5, Z6, Z8, Z10, Z11, Z13, and Z16.

6. A device for detecting a degree of benignity/malignancy of a thyroid tumor, wherein the device uses the model of any of claims 1-5 and comprises:

(1) a sampling unit for obtaining a test sample;
(2) a probe designing unit for designing a primer specific to the sequence of the imprinted gene, wherein the primer serves as a probe;
(3) a detection unit for performing in-situ hybridization between the probe designed by the probe designing unit and the test sample; and
(4) an analysis unit for analyzing expression of the imprinted gene through microscopic imaging;
wherein the analysis unit calculates and grades, via the model of any of claims 1-5, the expressed quantity of the imprinted gene with a loss of imprinting, the expressed quantity of the imprinted gene with a copy number variation, and the total expressed quantity of the imprinted gene, and diagnoses the degree of benignity/malignancy of the thyroid tumor according to grading results of the expressed quantities of the imprinted gene with, respectively, a loss of imprinting and a copy number variation.

7. A method for detecting a degree of benignity/malignancy of a thyroid tumor, wherein the method uses the model of any of claims 1-5 or the device of claim 6 and comprises the steps of:

(1) obtaining a test sample;
(2) designing a primer specific to the sequence of the imprinted gene, wherein the primer serves as a probe;
(3) performing in-situ hybridization between the probe designed in step (2) and the test sample; and
(4) analyzing expression of the imprinted gene through microscopic imaging so as to diagnose the degree of benignity/malignancy of the thyroid tumor;

wherein step (4) comprises calculating and grading, via the model of any of claims 1-5, the expressed quantity of the imprinted gene with a loss of imprinting, the expressed quantity of the imprinted gene with a copy number variation, and the total expressed quantity of the imprinted gene, and diagnosing the degree of benignity/malignancy of the thyroid tumor according to grading results of the expressed quantities of the imprinted gene with, respectively, a loss of imprinting and a copy number variation;
optionally wherein the test sample in step (1) is tissue and/or cells obtained from a human, optionally wherein the test sample is a paraffin section of a tissue and/or a cell smear prepared by a thyroid needle biopsy.

8. The method of claim 7, wherein the in-situ hybridization uses an RNAscope in-situ hybridization method, optionally wherein the RNAscope in-situ hybridization method uses a single- or multiple-channel chromogenic reagent kit or a single- or multiple-channel fluorescent reagent kit, preferably a single-channel red/brown chromogenic reagent kit or a multiple-channel fluorescent reagent kit.

9. The method of claim 7 or 8, wherein the degree of benignity/malignancy of the thyroid tumor is classified as one of the following: benign tumor, potential thyroid cancer, early-stage thyroid cancer, intermediate-stage thyroid cancer, and advanced thyroid cancer.

10. The method of any of claims 7-9, wherein the degree of benignity/malignancy of the thyroid tumor is diagnosed as benign tumor when the expressed quantities of each of the imprinted genes Z1, Z3, Z4, Z5, Z6, Z8, Z10, Z11, Z13, and Z16 with, respectively, a loss of imprinting and a copy number variation are both graded as Grade 0; or when the expressed quantity of not more than one of the imprinted genes Z1, Z3, Z4, Z5, Z6, Z8, Z10, Z11, Z13, and Z16 with a loss of imprinting is graded as Grade I, and the expressed quantity of not more than one of the imprinted genes Z1, Z3, Z4, Z5, Z6, Z8, Z10, Z11, Z13, and Z16 with a copy number variation is graded as Grade I;

the degree of benignity/malignancy of the thyroid tumor is diagnosed as potential thyroid cancer when the expressed quantities of at least two of the imprinted genes Z1, Z3, Z4, Z5, Z6, Z8, Z10, Z11, Z13, and Z16 with a loss of imprinting are graded as Grade I; or when the expressed quantities of at least two of the imprinted genes Z1, Z3, Z4, Z5, Z6, Z8, Z10, Z11, Z13, and Z16 with a copy number variation are graded as Grade I; or when the expressed quantity of not more than one of the imprinted genes Z1, Z3, Z4, Z5, Z6, Z8, Z10, Z11, Z13, and Z16 with a loss of imprinting is graded as Grade II, and the expressed quantity of not more than one of the imprinted genes Z1, Z3, Z4, Z5, Z6, Z8, Z10, Z11, Z13, and Z16 with a copy number variation is graded as Grade II;
the degree of benignity/malignancy of the thyroid tumor is diagnosed as early-stage thyroid cancer when the expressed quantities of at least two of the imprinted genes Z1, Z3, Z4, Z5, Z6, Z8, Z10, Z11, Z13, and Z16 with a loss of imprinting are graded as Grade II; or when the expressed quantities of at least two of the imprinted genes Z1, Z3, Z4, Z5, Z6, Z8, Z10, Z11, Z13, and Z16 with a copy number variation are graded as Grade II; or when the expressed quantity of not more than one of the imprinted genes Z1, Z3, Z4, Z5, Z6, Z8, Z10, Z11, Z13, and Z16 with a loss of imprinting is graded as Grade III, and the expressed quantity of not more than one of the imprinted genes Z1, Z3, Z4, Z5, Z6, Z8, Z10, Z11, Z13, and Z16 with a copy number variation is graded as Grade III;
the degree of benignity/malignancy of the thyroid tumor is diagnosed as intermediate-stage thyroid cancer when the expressed quantities of at least two of the imprinted genes Z1, Z3, Z4, Z5, Z6, Z8, Z10, Z11, Z13, and Z16 with a loss of imprinting are graded as Grade III; or when the expressed quantities of at least two of the imprinted genes Z1, Z3, Z4, Z5, Z6, Z8, Z10, Z11, Z13, and Z16 with a copy number variation are graded as Grade III; or when the expressed quantity of not more than one of the imprinted genes Z1, Z3, Z4, Z5, Z6, Z8, Z10, Z11, Z13, and Z16 with a loss of imprinting is graded as Grade IV, and the expressed quantity of not more than one of the imprinted genes Z1, Z3, Z4, Z5, Z6, Z8, Z10, Z11, Z13, and Z16 with a copy number variation is graded as Grade IV; and
the degree of benignity/malignancy of the thyroid tumor is diagnosed as advanced thyroid cancer when the expressed quantities of at least two of the imprinted genes Z1, Z3, Z4, Z5, Z6, Z8, Z10, Z11, Z13, and Z16 with a loss of imprinting are graded as Grade IV; or when the expressed quantities of at least two of the imprinted genes Z1, Z3, Z4, Z5, Z6, Z8, Z10, Z11, Z13, and Z16 with a copy number variation are graded as Grade IV.

11. A use of the model of any of claims 1-5 and/or the device of claim 6 in detecting a thyroid tumor.

12. The use of claim 11, wherein a degree of benignity/malignancy of the thyroid tumor is classified as one of the following: benign tumor, potential thyroid cancer, early-stage thyroid cancer, intermediate-stage thyroid cancer, and advanced thyroid cancer.

13. The use of claim 11 or 12, wherein a degree of benignity/malignancy of the thyroid tumor is diagnosed as benign tumor when the expressed quantities of each of the imprinted genes Z1, Z3, Z4, Z5, Z6, Z8, Z10, Z11, Z13, and Z16 with, respectively, a loss of imprinting and a copy number variation are both graded as Grade 0; or when the expressed quantity of not more than one of the imprinted genes Z1, Z3, Z4, Z5, Z6, Z8, Z10, Z11, Z13, and Z16 with a loss of imprinting is graded as Grade I, and the expressed quantity of not more than one of the imprinted genes Z1, Z3, Z4, Z5, Z6, Z8, Z10, Z11, Z13, and Z16 with a copy number variation is graded as Grade I;

the degree of benignity/malignancy of the thyroid tumor is diagnosed as potential thyroid cancer when the expressed quantities of at least two of the imprinted genes Z1, Z3, Z4, Z5, Z6, Z8, Z10, Z11, Z13, and Z16 with a loss of imprinting are graded as Grade I; or when the expressed quantities of at least two of the imprinted genes Z1, Z3, Z4, Z5, Z6, Z8, Z10, Z11, Z13, and Z16 with a copy number variation are graded as Grade I; or when the expressed quantity of not more than one of the imprinted genes Z1, Z3, Z4, Z5, Z6, Z8, Z10, Z11, Z13, and Z16 with a loss of imprinting is graded as Grade II, and the expressed quantity of not more than one of the imprinted genes Z1, Z3, Z4, Z5, Z6, Z8, Z10, Z11, Z13, and Z16 with a copy number variation is graded as Grade II;

the degree of benignity/malignancy of the thyroid tumor is diagnosed as early-stage thyroid cancer when the expressed quantities of at least two of the imprinted genes Z1, Z3, Z4, Z5, Z6, Z8, Z10, Z11, Z13, and Z16 with a loss of imprinting are graded as Grade II; or when the expressed quantities of at least two of the imprinted genes Z1, Z3, Z4, Z5, Z6, Z8, Z10, Z11, Z13, and Z16 with a copy number variation are graded as Grade II; or when the expressed quantity of not more than one of the imprinted genes Z1, Z3, Z4, Z5, Z6, Z8, Z10, Z11, Z13, and Z16 with a loss of imprinting is graded as Grade III, and the expressed quantity of not more than one of the imprinted genes Z1, Z3, Z4, Z5, Z6, Z8, Z10, Z11, Z13, and Z16 with a copy number variation is graded as Grade III;

the degree of benignity/malignancy of the thyroid tumor is diagnosed as intermediate-stage thyroid cancer when the expressed quantities of at least two of the imprinted genes Z1, Z3, Z4, Z5, Z6, Z8, Z10, Z11, Z13, and Z16 with a loss of imprinting are graded as Grade III; or when the expressed quantities of at least two of the imprinted genes Z1, Z3, Z4, Z5, Z6, Z8, Z10, Z11, Z13, and Z16 with a copy number variation are graded as Grade III; or when the expressed quantity of not more than one of the imprinted genes Z1, Z3, Z4, Z5, Z6, Z8, Z10, Z11, Z13, and Z16 with a loss of imprinting is graded as Grade IV, and the expressed quantity of not more than one of the imprinted genes Z1, Z3, Z4, Z5, Z6, Z8, Z10, Z11, Z13, and Z16 with a copy number variation is graded as Grade IV; and

the degree of benignity/malignancy of the thyroid tumor is diagnosed as advanced thyroid cancer when the expressed quantities of at least two of the imprinted genes Z1, Z3, Z4, Z5, Z6, Z8, Z10, Z11, Z13, and Z16 with a loss of imprinting are graded as Grade IV; or when the expressed quantities of at least two of the imprinted genes Z1, Z3, Z4, Z5, Z6, Z8, Z10, Z11, Z13, and Z16 with a copy number variation are graded as Grade IV.

**Patentansprüche**

1. Ein Gradierungsmodell für geprägte Gene, das auf einen Schilddrüsentumor anwendbar ist, wobei das Modell konfiguriert ist, Veränderungen in den folgenden exprimieten Mengen eines geprägten Gens im Schilddrüsentumor zu berechnen und einen Expressionsstatus des geprägten Gens dementsprechend zu gradieren: einer exprimierten Gesamtmenge des geprägten Gens, einer exprimierten Menge des geprägten Gens mit einem Verlust der Prägung und einer exprimierten Menge des geprägten Gens mit einer Kopienanzahlvariation;

wobei das geprägte Gen irgendeines, oder eine Kombination von mindestens zwei, von Z1, Z11 und Z16 ist, wobei das geprägte Gen Z1 Gnas ist, das geprägte Gen Z11 Grb10 ist, und das geprägte Gen Z16 Snrpn/Snurf ist; wobei die exprimierte Gesamtmenge des geprägten Gens, die exprimierte Menge des geprägten Gens mit einem Verlust der Prägung und die exprimierte Menge des geprägten Gens mit einer Kopienanzahlvariation nach den folgenden Formeln berechnet werden:

die exprimierte Gesamtmenge des geprägten Gens = (b+c+d)/(a+b+c+d)×100%;

eine exprimierte Menge des geprägten Gens, das normal ist = b/(b+c+d)×100%;

die exprimierte Menge des geprägten Gens mit einem Verlust der Prägung (LOI) = c/(b+c+d)×100%; und die exprimierte Menge des geprägten Gens mit einer Kopienanzahlvariation (CNV) = d/(b+c+d)×100%;

wobei a die Anzahl der Zellen ist, die, nach Färbung mit Hämatoxylin, keine Markierung im Kern jeder Zelle zeigen, was bedeutet, dass das geprägte Gen im Kern jeder Zelle nicht exprimiert ist; b die Anzahl von Zellen ist, die, nach Färbung mit Hämatoxylin, eine rote/braune Markierung im Kern jeder Zelle zeigen, was bedeutet, dass das geprägte Gen im Kern jeder Zelle vorliegt; c die Anzahl von Zellen ist, die, nach Färbung mit Hämatoxylin, zwei rote/braune Markierungen im Kern jeder Zelle zeigen, was bedeutet, dass das geprägte Gen im Kern jeder Zelle von einem Verlust der Prägung betroffen ist; und d die Anzahl von Zellen ist, die, nach Färbung mit Hämatoxylin, mehr als zwei rote/braune Markierungen im Kern jeder Zelle zeigen, was bedeutet, dass das geprägte Gen im Kern jeder Zelle eine Kopienanzahlvariation hat; und
wobei die exprimierte Menge des geprägten Gens mit einem Verlust der Prägung, die exprimierte Menge des geprägten Gens mit einer Kopienanzahlvariation und die exprimierte Gesamtmenge des geprägten Gens jeweils als einer von fünf verschiedenen Graden gradiert werden;
die exprimierte Menge jedes der geprägten Gene Z1 und Z16 mit einem Verlust der Prägung, die exprimierte Menge jedes der geprägten Gene Z1 und Z16 mit einer Kopienanzahlvariation und die exprimierte Gesamtmenge jedes der geprägten Gene Z1 und Z16 in die folgenden fünf Grade gradiert werden:
Grad 0: anwendbar, wenn irgendeine oder mindestens zwei der folgenden Bedingungen erfüllt sind: die exprimierte Menge eines der geprägten Gene Z1 und Z16 mit einem Verlust der Prägung ist weniger als 12%, die exprimierte Menge eines der geprägten Gene Z1 und Z16 mit einer Kopienanzahlvariation ist weniger als 1%, und die exprimierte Gesamtmenge eines der geprägten Gene Z1 und Z16 ist weniger als 25%;
Grad I: anwendbar, wenn irgendeine oder mindestens zwei der folgenden Bedingungen erfüllt sind: die exprimierte Menge eines der geprägten Gene Z1 und Z16 mit einem Verlust der Prägung ist 12%-20%, die exprimierte Menge eines der geprägten Gene Z1 und Z16 mit einer Kopienanzahlvariation ist 1%-2%, und die exprimierte Gesamtmenge eines der geprägten Gene Z1 und Z16 ist 25%-35%;
Grad II: anwendbar, wenn irgendeine oder mindestens zwei der folgenden Bedingungen erfüllt sind: die exprimierte Menge eines der geprägten Gene Z1 und Z16 mit einem Verlust der Prägung ist 20%-25%, die exprimierte Menge eines der geprägten Gene Z1 und Z16 mit einer Kopienanzahlvariation ist 2%-3%, und die exprimierte Gesamtmenge eines der geprägten Gene Z1 und Z16 ist 35%-45%;
Grad III: anwendbar, wenn irgendeine oder mindestens zwei der folgenden Bedingungen erfüllt sind: die exprimierte Menge eines der geprägten Gene Z1 und Z16 mit einem Verlust der Prägung ist 25%-30%, die exprimierte Menge eines der geprägten Gene Z1 und Z16 mit einer Kopienanzahlvariation ist 3%-5%, und die exprimierte Gesamtmenge eines der geprägten Gene Z1 und Z16 ist 45%-60%;
Grad IV: anwendbar, wenn irgendeine oder mindestens zwei der folgenden Bedingungen erfüllt sind: die exprimierte Menge eines der geprägten Gene Z1 und Z16 mit einem Verlust der Prägung ist größer als 30%, die exprimierte Menge eines der geprägten Gene Z1 und Z16 mit einer Kopienanzahlvariation ist größer als 5%, und die exprimierte Gesamtmenge eines der geprägten Gene Z1 und Z16 ist größer als 60%; und
die exprimierte Menge des geprägten Gens Z11 mit einem Verlust der Prägung, die exprimierte Menge des geprägten Gens Z11 mit einer Kopienanzahlvariation und die exprimierte Gesamtmenge des geprägten Gens Z11 in die folgenden fünf Grade gradiert werden:

Grad 0: anwendbar, wenn irgendeine oder mindestens zwei der folgenden Bedingungen erfüllt sind: die exprimierte Menge des geprägten Gens Z11 mit einem Verlust der Prägung ist weniger als 10%, die exprimierte Menge des geprägten Gens Z11 mit einer Kopienanzahlvariation ist weniger als 0,5%, und die exprimierte Gesamtmenge des geprägten Gens Z1 und Z16 ist weniger als 15%;
Grad I: anwendbar, wenn irgendeine oder mindestens zwei der folgenden Bedingungen erfüllt sind: die exprimierte Menge des geprägten Gens Z11 mit einem Verlust der Prägung ist 10%-15%, die exprimierte Menge des geprägten Gens Z11 mit einer Kopienanzahlvariation ist 0,5%-1%, und die exprimierte Gesamtmenge des geprägten Gens Z11 ist 15%-20%;
Grad II: anwendbar, wenn irgendeine oder mindestens zwei der folgenden Bedingungen erfüllt sind: die exprimierte Menge des geprägten Gens Z11 mit einem Verlust der Prägung ist 15%-20%, die exprimierte Menge des geprägten Gens Z11 mit einer Kopienanzahlvariation ist 1%-2%, und die exprimierte Gesamtmenge des geprägten Gens Z11 ist 20%-30%;

Grad III: anwendbar, wenn irgendeine oder mindestens zwei der folgenden Bedingungen erfüllt sind: die exprimierte Menge des geprägten Gens Z11 mit einem Verlust der Prägung ist 20%-25%, die exprimierte Menge des geprägten Gens Z11 mit einer Kopienanzahlvariation ist 2%-3%, und die exprimierte Gesamtmenge des geprägten Gens Z11 ist 30%-40%;

Grad IV: anwendbar, wenn irgendeine oder mindestens zwei der folgenden Bedingungen erfüllt sind: die exprimierte Menge des geprägten Gens Z11 mit einem Verlust der Prägung ist größer als 25%, die exprimierte Menge des geprägten Gens Z11 mit einer Kopienanzahlvariation ist größer als 3%, und die exprimierte Gesamtmenge des geprägten Gens Z11 ist größer als 40%.

2. Das Modell aus Anspruch 1, wobei das Modell die exprimierten Mengen irgendeines der gerpägten Gene Z1, Z11 und Z16 berechnet, vorzugsweise der geprägten Gene Z1 oder Z16, und bevorzugter des geprägten Gens Z1.

3. Das Modell aus Anspruch 1 oder 2, wobei das Modell die exprimierten Mengen einer Kombination irgendwelcher zwei der geprägten Gene Z1, Z11 und Z16 berechnet, bevorzugt einer Kombination der geprägten Gene Z1 und Z16 oder einer Kombination der geprägten Gene Z1 und Z11.

4. Das Modell aus irgendeinem der Ansprüche 1-3, wobei das geprägte Gen weiter eines, oder eine Kombination von mindestens zwei, von Z3, Z4, Z5, Z6, Z8, Z10 und Z13 umfasst, wobei das geürägte Gen Z3 Peg10 ist, das geprägte Gen Z4 Igf2r ist, das geprägte Gen Z5 Mest ist, das geprägte Gen Z6 Plagl1 ist, das geprägte Gen Z8 Dcn ist, das geprägte Gen Z10 Gatm ist, und das geprägte Gen Z13 Sgce ist;

die exprimierte Menge jedes der geprägten Gene Z3, Z4, Z5, Z6, Z8, Z10 und Z13 mit einem Verlust der Prägung, die exprimierte Menge jedes der geprägten Gene Z3, Z4, Z5, Z6, Z8, Z10 und Z13 mit einer Kopienanzahlvariation und die exprimierte Gesamtmenge jedes der geprägten Gene Z3, Z4, Z5, Z6, Z8, Z10 und Z13 in die folgenden fünf Grade gradiert werden:

Grad 0: anwendbar, wenn irgendeine oder mindestens zwei der folgenden Bedingungen erfüllt sind: die exprimierte Menge eines der geprägten Gene Z3, Z4, Z5, Z6, Z8, Z10 und Z13 mit einem Verlust der Prägung ist weniger als 10%, die exprimierte Menge eines der geprägten Gene Z3, Z4, Z5, Z6, Z8, Z10 und Z13 mit einer Kopienanzahlvariation ist weniger als 0,5%, und die exprimierte Gesamtmenge eines der geprägten Gene Z3, Z4, Z5, Z6, Z8, Z10 und Z13 ist weniger als 15%;

Grad I: anwendbar, wenn irgendeine oder mindestens zwei der folgenden Bedingungen erfüllt sind: die exprimierte Menge eines der geprägten Gene Z3, Z4, Z5, Z6, Z8, Z10 und Z13 mit einem Verlust der Prägung ist 10%-15%, die exprimierte Menge eines der geprägten Gene Z3, Z4, Z5, Z6, Z8, Z10 und Z13 mit einer Kopienanzahlvariation ist 0,5%-1%, und die exprimierte Gesamtmenge eines der geprägten Gene Z3, Z4, Z5, Z6, Z8, Z10 und Z13 ist 15%-20%;

Grad II: anwendbar, wenn irgendeine oder mindestens zwei der folgenden Bedingungen erfüllt sind: die exprimierte Menge eines der geprägten Gene Z3, Z4, Z5, Z6, Z8, Z10 und Z13 mit einem Verlust der Prägung ist 15%-20%, die exprimierte Menge eines der geprägten Gene Z3, Z4, Z5, Z6, Z8, Z10 und Z13 mit einer Kopienanzahlvariation ist 1%-2%, und die exprimierte Gesamtmenge eines der geprägten Gene Z3, Z4, Z5, Z6, Z8, Z10 und Z13 ist 20%-30%;

Grad III: anwendbar, wenn irgendeine oder mindestens zwei der folgenden Bedingungen erfüllt sind: die exprimierte Menge eines der geprägten Gene Z3, Z4, Z5, Z6, Z8, Z10 und Z13 mit einem Verlust der Prägung ist 20%-25%, die exprimierte Menge eines der geprägten Gene Z3, Z4, Z5, Z6, Z8, Z10 und Z13 mit einer Kopienanzahlvariation ist 2%-3%, und die exprimierte Gesamtmenge eines der geprägten Gene Z3, Z4, Z5, Z6, Z8, Z10 und Z13 ist 30%-40%;

Grad IV: anwendbar, wenn irgendeine oder mindestens zwei der folgenden Bedingungen erfüllt sind: die exprimierte Menge eines der geprägten Gene Z3, Z4, Z5, Z6, Z8, Z10 und Z13 mit einem Verlust der Prägung ist größer als 25%, die exprimierte Menge eines der geprägten Gene Z3, Z4, Z5, Z6, Z8, Z10 und Z13 mit einer Kopienanzahlvariation ist größer als 3%, und die exprimierte Gesamtmenge eines der geprägten Gene Z3, Z4, Z5, Z6, Z8, Z10 und Z13 ist größer als 40%.

5. Das Modell aus irgendeinem der Ansprüche 1-4, wobei das Modell die exprimierten Mengen einer Kombination der zehn geprägten Gene Z1, Z3, Z4, Z5, Z6, Z8, Z10, Z11, Z13 und Z16 berechnet.

6. Eine Vorrichtung zum Nachweis eines Grades der Gutartigkeit/Bösartigkeit eines Schilddrüsentumors, wobei die Vorrichtung das Modell aus irgendeinem der Ansprüche 1-5 verwendet und Folgendes umfasst:

(1) eine Probeneinheit zum Erhalten einer Testprobe;

(2) eine Sondenentwurfseinheit zum Entwerfen eines Primers, der spezifisch für die Sequenz des geprägten Gens ist, wobei der Primer als Sonde dient;

(3) eine Detektionseinheit zum Durchführen von in-situ Hybridisierung zwischen der von der Sondenentwurfseinheit entworfenen Sonde und der Testprobe; und

(4) eine Analyseeinheit zum Analysieren der Expression der geprägten Gene durch mikroskopische Bildgebung; wobei die Analyseeinheit mittels des Modells aus irgendeinem der Ansprüche 1-5 die exprimierte Menge des geprägten Gens mit einem Verlust der Prägung, die exprimierte Menge des geprägten Gens mit einer Kopienanzahlvariation und die exprimierte Gesamtmenge des geprägten Gens berechnet und gradiert, und den Grad der Gutartigkeit/Bösartigkeit des Schilddrüsentumors gemäß den Gradierungsergebnissen der exprimierten Mengen des geprägten Gens einem Verlust der Prägung bzw. einer Kopienanzahlvariation diagnostiziert.

7. Ein Verfahren zum Nachweis eines Grades der Gutartigkeit/Bösartigkeit eines Schilddrüsentumors, wobei das Verfahren das Modell aus irgendeinem der Ansprüche 1-5 oder die Vorrichtung aus Anspruch 6 verwendet und folgende Schritte umfasst:

(1) Erhalten einer Testprobe;

(2) Entwerfen eines Primers, der spezifisch für die Sequenz des geprägten Gens ist, wobei der Primer als Sonde dient;

(3) Durchführen von in-situ Hybridisierung zwischen der von der in Schritt (2) entworfenen Sonde und der Testprobe; und

(4) Analysieren der Expression der geprägten Gene durch mikroskopische Bildgebung, um den Grad der Gutartigkeit/Bösartigkeit des Schilddrüsentumors zu diagnostizieren;

wobei Schritt (4) das Berechnen und Gradieren, mitteld des Modells aus irgendeinem der Ansprüche 1-5, der exprimierten Menge des geprägten Gens mit einem Verlust der Prägung, der exprimierten Menge des geprägten Gens mit einer Kopienanzahlvariation und der exprimierten Gesamtmenge des geprägten Gens, und das Diagnostizieren des Grades der Gutartigkeit/Bösartigkeit des Schilddrüsentumors gemäß den Gradierungsergebnissen der exprimierten Mengen des geprägten Gens einem Verlust der Prägung bzw. einer Kopienanzahlvariation umfasst;

optional wobei die Testprobe in Schritt (1) von einem Menschen erhaltenes Gewebe und/oder Zellen ist, optional wobei die Testprobe ein Paraffinschnitt eines Gewebes und/oder ein durch Schilddrüsen-Nadelbiopsie hergestellter Zellabstrich ist.

8. Das Verfahren aus Anspruch 7, wobei die in-situ Hybridisierung ein RNAscope in-situ Hybridisierungsverfagren verwendet, optional wobei das RNAscope in-situ Hybridisierungsverfahren ein Kit mit ein- oder mehrkanaligen chromogenen Reagsnzien oder ein Kit mirt ein- oder mehrkanaligen fluoreszierenden Reagenzien verwendet, vorzugsweise ein Kit mit einkanaligem rot/braunem chromogenem Reagens oder ein Kit mit mehrkanaligem fluorsezierendem Reagens.

9. Das Verfahren aus Anspruch 7 oder 8, wobei der Grad der Gutartigkeit/Bösartigkeit des Schilddrüsentumors als eines der Folgenden klassifiziert wird: gutartiger Tumor, potentieller Schilddrüsenkrebs, Schilddrüsenkrebs im frühen Stadium, Schilddrüsenkrebs im Übergangsstadium, Schilddrüsenkrebs im fortgeschrittenen Stadium.

10. Das Verfahren aus irgendeinem der Ansprüche 7-9, wobei der Grad der Gutartigkeit/Bösartigkeit des Schilddrüsentumors als ein gutartiger Tumor diagnostiziert wird, wenn die exprimierten Mengen jedes der geprägten Gene Z1, Z3, Z4, Z5, Z6, Z8, Z10, Z11, Z13 und Z16 mit einem Verlust der Prägung bzw. einer Kopienanzahlvariation beide als Grad 0 gradiert werden; oder wenn die exprimierte Menge von nicht mehr als einem der geprägten Gene Z1, Z3, Z4, Z5, Z6, Z8, Z10, Z11, Z13 und Z16 mit einem Verlust der Prägung als Grad I gradiert wird und die exprimierte Menge von nicht mehr als einem der geprägten Gene Z1, Z3, Z4, Z5, Z6, Z8, Z10, Z11, Z13 und Z16 mit einer Kopienanzahlvariation als Grad I gradiert wird;

der Grad der Gutartigkeit/Bösartigkeit des Schilddrüsentumors als ein potentieller Schilddrüsenkrebs diagnostiziert wird, wenn die exprimierten Mengen von mindestens zwei der geprägten Gene Z1, Z3, Z4, Z5, Z6, Z8, Z10, Z11, Z13 und Z16 mit einem Verlust der Prägung als Grad I gradiert werden; oder wenn die exprimierten Menge von mindestens zwei der geprägten Gene Z1, Z3, Z4, Z5, Z6, Z8, Z10, Z11, Z13 und Z16 mit einer Kopienanzahlvariation als Grad I gradiert werden; oder wenn die exprimierte Menge von nicht mehr als einem der geprägten Gene Z1, Z3, Z4, Z5, Z6, Z8, Z10, Z11, Z13 und Z16 mit einem Verlust der Prägung als Grad II gradiert wird und die exprimierte Menge von nicht mehr als einem der geprägten Gene Z1, Z3, Z4, Z5, Z6, Z8, Z10, Z11, Z13 und Z16 mit einer Kopienanzahlvariation als Grad II gradiert wird;

der Grad der Gutartigkeit/Bösartigkeit des Schilddrüsentumors als ein Schilddrüsenkrebs im frühen Stadium diagnostiziert wird, wenn die exprimierten Mengen von mindestens zwei der geprägten Gene Z1, Z3, Z4, Z5, Z6, Z8, Z10, Z11, Z13 und Z16 mit einem Verlust der Prägung als Grad II gradiert werden; oder wenn die exprimierten Menge von mindestens zwei der geprägten Gene Z1, Z3, Z4, Z5, Z6, Z8, Z10, Z11, Z13 und Z16 mit einer Kopienanzahlvariation als Grad II gradiert werden; oder wenn die exprimierte Menge von nicht mehr als einem der geprägten Gene Z1, Z3, Z4, Z5, Z6, Z8, Z10, Z11, Z13 und Z16 mit einem Verlust der Prägung als Grad III gradiert wird und die exprimierte Menge von nicht mehr als einem der geprägten Gene Z1, Z3, Z4, Z5, Z6, Z8, Z10, Z11, Z13 und Z16 mit einer Kopienanzahlvariation als Grad III gradiert wird;

der Grad der Gutartigkeit/Bösartigkeit des Schilddrüsentumors als ein Schilddrüsenkrebs im Übergangsstadium diagnostiziert wird, wenn die exprimierten Mengen von mindestens zwei der geprägten Gene Z1, Z3, Z4, Z5, Z6, Z8, Z10, Z11, Z13 und Z16 mit einem Verlust der Prägung als Grad III gradiert werden; oder wenn die exprimierten Menge von mindestens zwei der geprägten Gene Z1, Z3, Z4, Z5, Z6, Z8, Z10, Z11, Z13 und Z16 mit einer Kopienanzahlvariation als Grad III gradiert werden; oder wenn die exprimierte Menge von nicht mehr als einem der geprägten Gene Z1, Z3, Z4, Z5, Z6, Z8, Z10, Z11, Z13 und Z16 mit einem Verlust der Prägung als Grad IV gradiert wird und die exprimierte Menge von nicht mehr als einem der geprägten Gene Z1, Z3, Z4, Z5, Z6, Z8, Z10, Z11, Z13 und Z16 mit einer Kopienanzahlvariation als Grad IV gradiert wird;

der Grad der Gutartigkeit/Bösartigkeit des Schilddrüsentumors als ein Schilddrüsenkrebs im fortgeschrittenen Stadium diagnostiziert wird, wenn die exprimierten Mengen von mindestens zwei der geprägten Gene Z1, Z3, Z4, Z5, Z6, Z8, Z10, Z11, Z13 und Z16 mit einem Verlust der Prägung als Grad IV gradiert werden; oder wenn die exprimierten Menge von mindestens zwei der geprägten Gene Z1, Z3, Z4, Z5, Z6, Z8, Z10, Z11, Z13 und Z16 mit einer Kopienanzahlvariation als Grad IV gradiert werden.

11. Eine Verwendung des Modells aus irgendeinem der Ansprüche 1-5 und/oder der Vorrichtung aus Anspruch 6 zum Nachweis eines Schilddrüsentumors.

12. Die Verwendung aus Anspruch 11, wobei der Grad der Gutartigkeit/Bösartigkeit des Schilddrüsentumors als eines der Folgenden klassifiziert wird: gutartiger Tumor, potentieller Schilddrüsenkrebs, Schilddrüsenkrebs im frühen Stadium, Schilddrüsenkrebs im Übergangsstadium, Schilddrüsenkrebs im fortgeschrittenen Stadium.

13. Die Verwendung aus Anspruch 11 oder 12, wobei der Grad der Gutartigkeit/Bösartigkeit des Schilddrüsentumors als ein gutartiger Tumor diagnostiziert wird, wenn die exprimierten Mengen jedes der geprägten Gene Z1, Z3, Z4, Z5, Z6, Z8, Z10, Z11, Z13 und Z16 mit einem Verlust der Prägung bzw. einer Kopienanzahlvariation beide als Grad 0 gradiert werden; oder wenn die exprimierte Menge von nicht mehr als einem der geprägten Gene Z1, Z3, Z4, Z5, Z6, Z8, Z10, Z11, Z13 und Z16 mit einem Verlust der Prägung als Grad I gradiert wird und die exprimierte Menge von nicht mehr als einem der geprägten Gene Z1, Z3, Z4, Z5, Z6, Z8, Z10, Z11, Z13 und Z16 mit einer Kopienanzahlvariation als Grad I gradiert wird;

der Grad der Gutartigkeit/Bösartigkeit des Schilddrüsentumors als ein potentieller Schilddrüsenkrebs diagnostiziert wird, wenn die exprimierten Mengen von mindestens zwei der geprägten Gene Z1, Z3, Z4, Z5, Z6, Z8, Z10, Z11, Z13 und Z16 mit einem Verlust der Prägung als Grad I gradiert werden; oder wenn die exprimierten Menge von mindestens zwei der geprägten Gene Z1, Z3, Z4, Z5, Z6, Z8, Z10, Z11, Z13 und Z16 mit einer Kopienanzahlvariation als Grad I gradiert werden; oder wenn die exprimierte Menge von nicht mehr als einem der geprägten Gene Z1, Z3, Z4, Z5, Z6, Z8, Z10, Z11, Z13 und Z16 mit einem Verlust der Prägung als Grad II gradiert wird und die exprimierte Menge von nicht mehr als einem der geprägten Gene Z1, Z3, Z4, Z5, Z6, Z8, Z10, Z11, Z13 und Z16 mit einer Kopienanzahlvariation als Grad II gradiert wird;

der Grad der Gutartigkeit/Bösartigkeit des Schilddrüsentumors als ein Schilddrüsenkrebs im frühen Stadium diagnostiziert wird, wenn die exprimierten Mengen von mindestens zwei der geprägten Gene Z1, Z3, Z4, Z5, Z6, Z8, Z10, Z11, Z13 und Z16 mit einem Verlust der Prägung als Grad II gradiert werden; oder wenn die exprimierten Menge von mindestens zwei der geprägten Gene Z1, Z3, Z4, Z5, Z6, Z8, Z10, Z11, Z13 und Z16 mit einer Kopienanzahlvariation als Grad II gradiert werden; oder wenn die exprimierte Menge von nicht mehr als einem der geprägten Gene Z1, Z3, Z4, Z5, Z6, Z8, Z10, Z11, Z13 und Z16 mit einem Verlust der Prägung als Grad III gradiert wird und die exprimierte Menge von nicht mehr als einem der geprägten Gene Z1, Z3, Z4, Z5, Z6, Z8, Z10, Z11, Z13 und Z16 mit einer Kopienanzahlvariation als Grad III gradiert wird;

der Grad der Gutartigkeit/Bösartigkeit des Schilddrüsentumors als ein Schilddrüsenkrebs im Übergangsstadium diagnostiziert wird, wenn die exprimierten Mengen von mindestens zwei der geprägten Gene Z1, Z3, Z4, Z5, Z6, Z8, Z10, Z11, Z13 und Z16 mit einem Verlust der Prägung als Grad III gradiert werden; oder wenn die exprimierten Menge von mindestens zwei der geprägten Gene Z1, Z3, Z4, Z5, Z6, Z8, Z10, Z11, Z13 und Z16 mit einer Kopienanzahlvariation als Grad III gradiert werden; oder wenn die exprimierte Menge von nicht mehr als einem der geprägten Gene Z1, Z3, Z4, Z5, Z6, Z8, Z10, Z11, Z13 und Z16 mit einem Verlust der Prägung als Grad IV gradiert wird und die exprimierte Menge von nicht mehr als einem der geprägten Gene Z1, Z3, Z4, Z5, Z6, Z8, Z10, Z11, Z13 und Z16 mit einer Kopienanzahlvariation als Grad IV gradiert wird;

der Grad der Gutartigkeit/Bösartigkeit des Schilddrüsentumors als ein Schilddrüsenkrebs im fortgeschrittenen Stadium diagnostiziert wird, wenn die exprimierten Mengen von mindestens zwei der geprägten Gene Z1, Z3, Z4, Z5, Z6, Z8, Z10, Z11, Z13 und Z16 mit einem Verlust der Prägung als Grad IV gradiert werden; oder wenn die exprimierten Menge von mindestens zwei der geprägten Gene Z1, Z3, Z4, Z5, Z6, Z8, Z10, Z11, Z13 und Z16 mit einer Kopienanzahlvariation als Grad IV gradiert werden.

## Revendications

1. Modèle de classification de gène soumis à empreinte applicable à une tumeur thyroïdienne, le modèle étant configuré pour calculer des changements des quantités exprimées suivantes d'un gène soumis à empreinte dans la tumeur thyroïdienne et classifier un état d'expression du gène soumis à empreinte en conséquence : une quantité exprimée totale du gène soumis à empreinte, une quantité exprimée du gène soumis à empreinte avec une perte d'empreinte, et une quantité exprimée du gène soumis à empreinte avec une variation du nombre de copies ;

dans lequel le gène soumis à empreinte est l'un quelconque, ou une combinaison d'au moins deux, parmi Z1, Z11 et Z16, le gène soumis à empreinte Z1 étant Gnas, le gène soumis à empreinte Z11 étant Grb10, et le gène soumis à empreinte Z16 étant Snrpn/Snurf ;

dans lequel la quantité exprimée totale du gène soumis à empreinte, la quantité exprimée du gène soumis à empreinte avec une perte d'empreinte, et la quantité exprimée du gène soumis à empreinte avec une variation du nombre de copies sont calculées par les formules suivantes :

la quantité exprimée totale du gène soumis à empreinte = $(b+c+d)/(a+b+c+d) \times 100\%$ ;

une quantité exprimée du gène soumis à empreinte qui est normale = $b/(b+c+d) \times 100\%$ ;

la quantité exprimée du gène soumis à empreinte avec une perte d'empreinte (LOI) = $c/(b+c+d) \times 100\%$ ; et

la quantité exprimée du gène soumis à empreinte avec une variation du nombre de copies (CNV) = $d/(b+c+d) \times 100\%$ ;

où a est le nombre de cellules qui, après avoir été colorées à l'hématoxyline, ne présentent aucune marque dans le noyau de chacune desdites cellules, ce qui signifie que le gène soumis à empreinte n'est pas exprimé dans le noyau de chacune desdites cellules ; b est le nombre de cellules qui, après avoir été colorées à l'hématoxyline, présentent une marque rouge/brune dans le noyau de chacune desdites cellules, ce qui signifie que le gène soumis à empreinte est présent dans le noyau de chacune desdites cellules ; c est le nombre de cellules qui, après avoir été colorées à l'hématoxyline, présentent deux marques rouges/brunes dans le noyau de chacune desdites cellules, ce qui signifie que le gène soumis à empreinte dans le noyau de chacune desdites cellules est affecté par une perte d'empreinte ; et d est le nombre de cellules qui, après avoir été colorées à l'hématoxyline, présentent plus de deux marques rouges/brunes dans le noyau de chacune desdites cellules, ce qui signifie que le gène soumis à empreinte dans le noyau de chacune desdites cellules présente une variation du nombre de copies ; et dans lequel la quantité exprimée du gène soumis à empreinte avec une perte d'empreinte, la quantité exprimée du gène soumis à empreinte avec une variation du nombre de copies, et la quantité exprimée totale du gène soumis à empreinte sont chacune classifiées selon l'un de cinq grades différents ;

la quantité exprimée de chacun des gènes soumis à empreinte Z1 et Z16 avec une perte d'empreinte, la quantité exprimée de chacun des gènes soumis à empreinte Z1 et Z16 avec une variation du nombre de copies, et la quantité exprimée totale de chacun des gènes soumis à empreinte Z1 et Z16 sont classifiées selon les cinq grades suivants :

Grade 0 : applicable lorsque l'une quelconque ou au moins deux des conditions suivantes sont satisfaites : la quantité exprimée d'un des gènes soumis à empreinte Z1 et Z16 avec une perte d'empreinte est inférieure à 12 %, la quantité exprimée d'un des gènes soumis à empreinte Z1 et Z16 avec une variation du nombre de copies est inférieure à 1 %, et la quantité exprimée totale d'un des gènes soumis à empreinte Z1 et Z16 est inférieure à 25 % ;

Grade I : applicable lorsque l'une quelconque ou au moins deux des conditions suivantes sont satisfaites : la quantité exprimée d'un des gènes soumis à empreinte Z1 et Z16 avec une perte d'empreinte est de 12 % à 20 %, la quantité exprimée d'un des gènes soumis à empreinte Z1 et Z16 avec une variation du nombre de copies est de 1 % à 2 %, et la quantité exprimée totale d'un des gènes soumis à empreinte Z1 et Z16 est de 25 % à 35 % ;

Grade II : applicable lorsque l'une quelconque ou au moins deux des conditions suivantes sont satisfaites : la quantité exprimée d'un des gènes soumis à empreinte Z1 et Z16 avec une perte d'empreinte est de 20 % à 25 %, la quantité exprimée d'un des gènes soumis à empreinte Z1 et Z16 avec une variation du nombre de copies est de 2 % à 3 %, et la quantité exprimée totale d'un des gènes soumis à empreinte Z1 et Z16 est de 35 % à 45 % ;

Grade III : applicable lorsque l'une quelconque ou au moins deux des conditions suivantes sont satisfaites : la quantité exprimée d'un des gènes soumis à empreinte Z1 et Z16 avec une perte d'empreinte est de 25 % à 30 %, la quantité exprimée d'un des gènes soumis à empreinte Z1 et Z16 avec une variation du nombre de copies est de 3 % à 5 %, et la quantité exprimée totale d'un des gènes soumis à empreinte Z1 et Z16 est de 45 % à 60 % ; et

Grade IV : applicable lorsque l'une quelconque ou au moins deux des conditions suivantes sont satisfaites : la quantité exprimée d'un des gènes soumis à empreinte Z1 et Z16 avec une perte d'empreinte est supérieure à 30 %, la quantité exprimée d'un des gènes soumis à empreinte Z1 et Z16 avec une variation du nombre de copies est supérieure à 5 %, et la quantité exprimée totale d'un des gènes soumis à empreinte Z1 et Z16 est supérieure à 60 % ; et

la quantité exprimée du gène soumis à empreinte Z11 avec une perte d'empreinte, la quantité exprimée du gène soumis à empreinte Z11 avec une variation du nombre de copies, et la quantité exprimée totale du gène soumis à empreinte Z11 sont classifiées selon les cinq grades suivants :

Grade 0 : applicable lorsque l'une quelconque ou au moins deux des conditions suivantes sont satisfaites : la quantité exprimée du gène Z11 soumis à empreinte avec une perte d'empreinte est inférieure à 10 %, la quantité exprimée du gène soumis à empreinte Z11 avec une variation du nombre de copies est inférieure à 0,5 %, et la quantité exprimée totale du gène soumis à empreinte Z11 est inférieure à 15 % ;

Grade I : applicable lorsque l'une quelconque ou au moins deux des conditions suivantes sont satisfaites : la quantité exprimée du gène soumis à empreinte Z11 avec une perte d'empreinte est de 10 % à 15 %, la quantité exprimée du gène soumis à empreinte Z11 avec une variation du nombre de copies est de 0,5 % à 1 %, et la quantité exprimée totale du gène soumis à empreinte Z11 est de 15 % à 20 % ;

Grade II : applicable lorsque l'une quelconque ou au moins deux des conditions suivantes sont satisfaites : la quantité exprimée du gène soumis à empreinte Z11 avec une perte d'empreinte est de 15 % à 20 %, la quantité exprimée du gène soumis à empreinte Z11 avec une variation du nombre de copies est de 1 % à 2 %, et la quantité exprimée totale du gène soumis à empreinte Z11 est de 20 % à 30 % ;

Grade III : applicable lorsque l'une quelconque ou au moins deux des conditions suivantes sont satisfaites : la quantité exprimée du gène soumis à empreinte Z11 avec une perte d'empreinte est de 20 % à 25 %, la quantité exprimée du gène soumis à empreinte Z11 avec une variation du nombre de copies est de 2 % à 3 %, et la quantité exprimée totale du gène soumis à empreinte Z11 est de 30 % à 40 % ;

Grade IV : applicable lorsque l'une quelconque ou au moins deux des conditions suivantes sont satisfaites : la quantité exprimée du gène soumis à empreinte Z11 avec une perte d'empreinte est supérieure à 25 %, la quantité exprimée du gène soumis à empreinte Z11 avec une variation du nombre de copies est supérieure à 3 %, et la quantité exprimée totale du gène soumis à empreinte Z11 est supérieure à 40 %.

2. Modèle selon la revendication 1, dans lequel le modèle calcule les quantités exprimées de l'un quelconque des gènes soumis à empreinte Z1, Z11 et Z16, de préférence le gène soumis à empreinte Z1 ou Z16, et plus préférentiellement le gène soumis à empreinte Z1.

3. Modèle selon la revendication 1 ou 2, dans lequel le modèle calcule les quantités exprimées d'une combinaison quelconque de deux des gènes soumis à empreinte Z1, Z11 et Z16, de préférence une combinaison des gènes soumis à empreinte Z1 et Z16 ou une combinaison des gènes soumis à empreinte Z1 et Z11.

4. Modèle selon l'une quelconque des revendications 1 à 3, dans lequel le gène soumis à empreinte comprend en outre l'un quelconque, ou une combinaison d'au moins deux, parmi Z3, Z4, Z5, Z6, Z8, Z10 et Z13, le gène soumis à empreinte Z3 étant Peg10, le gène soumis à empreinte Z4 étant Igf2r, le gène soumis à empreinte Z5 étant Mest, le gène soumis à empreinte Z6 étant Plagl1, le gène soumis à empreinte Z8 étant Dcn, le gène soumis à empreinte Z10 étant Gatm, et le gène soumis à empreinte Z13 étant Sgce ;

la quantité exprimée de chacun des gènes soumis à empreinte Z3, Z4, Z5, Z6, Z8, Z10 et Z13 avec une perte d'empreinte, la quantité exprimée de chacun des gènes soumis à empreinte Z3, Z4, Z5, Z6, Z8, Z10 et Z13 avec une variation du nombre de copies, et la quantité exprimée totale de chacun des gènes soumis à empreinte Z3, Z4, Z5, Z6, Z8, Z10 et Z13 sont classifiés selon les cinq grades suivants :

Grade 0 : applicable lorsque l'une quelconque ou au moins deux des conditions suivantes sont satisfaites : la quantité exprimée d'un des gènes soumis à empreinte Z3, Z4, Z5, Z6, Z8, Z10 et Z13 avec une perte d'empreinte est inférieure à 10 %, la quantité exprimée d'un des gènes soumis à empreinte Z3, Z4, Z5, Z6, Z8, Z10 et Z13 avec une variation du nombre de copies est inférieure à 0,5 %, et la quantité exprimée totale d'un des gènes soumis à empreinte Z3, Z4, Z5, Z6, Z8, Z10 et Z13 est inférieure à 15 % ;

Grade I : applicable lorsque l'une quelconque ou au moins deux des conditions suivantes sont satisfaites : la quantité exprimée d'un des gènes soumis à empreinte Z3, Z4, Z5, Z6, Z8, Z10 et Z13 avec une perte d'empreinte est de 10 % à 15 %, la quantité exprimée d'un des gènes soumis à empreinte Z3, Z4, Z5, Z6, Z8, Z10, et Z13 avec une variation du nombre de copies est de 0,5 % à 1 %, et la quantité exprimée totale de l'un des gènes soumis à empreinte Z3, Z4, Z5, Z6, Z8, Z10, et Z13 est de 15 % à 20 % ;

Grade II : applicable lorsque l'une quelconque ou au moins deux des conditions suivantes sont satisfaites : la quantité exprimée d'un des gènes soumis à empreinte Z3, Z4, Z5, Z6, Z8, Z10 et Z13 avec une perte d'empreinte est de 15 % à 20 %, la quantité exprimée d'un des gènes soumis à empreinte Z3, Z4, Z5, Z6, Z8, Z10, et Z13 avec une variation du nombre de copies est de 1 % à 2 %, et la quantité exprimée totale de l'un des gènes soumis à empreinte Z3, Z4, Z5, Z6, Z8, Z10, et Z13 est de 20 % à 30 % ;

Grade III : applicable lorsque l'une quelconque ou au moins deux des conditions suivantes sont satisfaites : la quantité exprimée d'un des gènes soumis à empreinte Z3, Z4, Z5, Z6, Z8, Z10 et Z13 avec une perte d'empreinte est de 20 % à 25 %, la quantité exprimée d'un des gènes soumis à empreinte Z3, Z4, Z5, Z6, Z8, Z10, et Z13 avec une variation du nombre de copies est de 2 % à 3 %, et la quantité exprimée totale de l'un des gènes soumis à empreinte Z3, Z4, Z5, Z6, Z8, Z10, et Z13 est de 30 % à 40 % ; et

Grade IV : applicable lorsque l'une quelconque ou au moins deux des conditions suivantes sont satisfaites : la quantité exprimée d'un des gènes soumis à empreinte Z3, Z4, Z5, Z6, Z8, Z10 et Z13 avec une perte d'empreinte est supérieure à 25 %, la quantité exprimée d'un des gènes soumis à empreinte Z3, Z4, Z5, Z6, Z8, Z10, et Z13 avec une variation du nombre de copies est supérieure à 3 %, et la quantité exprimée totale d'un des gènes soumis à empreinte Z3, Z4, Z5, Z6, Z8, Z10, et Z13 est supérieure à 40 %.

5. Modèle selon l'une quelconque des revendications 1 à 4, dans lequel le modèle calcule les quantités exprimées d'une combinaison des dix gènes soumis à empreinte Z1, Z3, Z4, Z5, Z6, Z8, Z10, Z11, Z13 et Z16.

6. Dispositif de détection d'un caractère bénin/malin d'une tumeur thyroïdienne, dans lequel le dispositif utilise le modèle selon l'une quelconque des revendications 1 à 5 et comprend :

(1) une unité d'échantillonnage pour obtenir un échantillon d'essai ;
(2) une unité de conception de sonde pour concevoir une amorce spécifique à la séquence du gène soumis à empreinte, dans lequel l'amorce sert de sonde ;
(3) une unité de détection pour réaliser une hybridation in situ entre la sonde conçue par l'unité de conception de sonde et l'échantillon d'essai ; et
(4) une unité d'analyse pour analyser l'expression du gène soumis à empreinte par imagerie microscopique ; dans lequel l'unité d'analyse calcule et classifie, par l'intermédiaire du modèle selon l'une quelconque des revendications 1 à 5, la quantité exprimée du gène soumis à empreinte avec une perte d'empreinte, la quantité exprimée du gène soumis à empreinte avec une variation du nombre de copies, et la quantité exprimée totale du gène soumis à empreinte, et diagnostique le caractère bénin/malin de la tumeur thyroïdienne en fonction des résultats de classification des quantités exprimées du gène soumis à empreinte avec, respectivement, une perte d'empreinte et une variation du nombre de copies.

7. Procédé de détection d'un caractère bénin/malin d'une tumeur thyroïdienne, dans lequel le procédé utilise le modèle selon l'une quelconque des revendications 1 à 5 ou le dispositif selon la revendication 6 et comprend les étapes suivantes :

(1) l'obtention d'un échantillon d'essai ;

(2) la conception d'une amorce spécifique à la séquence du gène soumis à empreinte, dans lequel l'amorce sert de sonde ;

(3) la réalisation d'une hybridation in situ entre la sonde conçue dans l'étape (2) et l'échantillon d'essai ; et

(4) l'analyse de l'expression du gène soumis à empreinte par imagerie microscopique de manière à diagnostiquer le caractère bénin/malin de la tumeur thyroïdienne ;

dans lequel l'étape (4) comprend le calcul et la classification, par l'intermédiaire du modèle selon l'une quelconque des revendications 1 à 5, de la quantité exprimée du gène soumis à empreinte avec une perte d'empreinte, de la quantité exprimée du gène soumis à empreinte avec une variation du nombre de copies, et de la quantité exprimée totale du gène soumis à empreinte, et le diagnostic du caractère bénin/malin de la tumeur thyroïdienne en fonction des résultats de classification des quantités exprimées du gène soumis à empreinte avec, respectivement, une perte d'empreinte et une variation du nombre de copies ; facultativement dans lequel l'échantillon d'essai dans l'étape (1) est un tissu et/ou des cellules prélevés sur un humain, facultativement dans lequel l'échantillon d'essai est une section de tissu dans la paraffine et/ou un frottis cellulaire préparé par biopsie à l'aiguille de la thyroïde.

8. Procédé selon la revendication 7, dans lequel l'hybridation in situ utilise un procédé d'hybridation in situ RNAscope, facultativement dans lequel le procédé d'hybridation in situ RNAscope utilise un kit de réactif chromogène monocanal ou multicanal ou un kit de réactif fluorescent monocanal ou multicanal, de préférence un kit de réactif chromogène rouge/brun monocanal ou un kit de réactif fluorescent multicanal.

9. Procédé selon la revendication 7 ou 8, dans lequel le caractère bénin/malin de la tumeur thyroïdienne est classifié comme l'un des suivants : tumeur bénigne, cancer thyroïdien potentiel, cancer thyroïdien de stade précoce, cancer thyroïdien de stade intermédiaire et cancer thyroïdien avancé.

10. Procédé selon l'une quelconque des revendications 7 à 9, dans lequel le caractère bénin/malin de la tumeur thyroïdienne est diagnostiqué une tumeur bénigne lorsque les quantités exprimées de chacun des gènes soumis à empreinte Z1, Z3, Z4, Z5, Z6, Z8, Z10, Z11, Z13 et Z16 avec, respectivement, une perte d'empreinte et une variation du nombre de copies sont toutes deux classifiées Grade 0 ; ou lorsque la quantité exprimée de pas plus d'un des gènes soumis à empreinte Z1, Z3, Z4, Z5, Z6, Z8, Z10, Z11, Z13, et Z16 avec une perte d'empreinte est classifiée Grade I, et que la quantité exprimée de pas plus d'un des gènes soumis à empreinte Z1, Z3, Z4, Z5, Z6, Z8, Z10, Z11, Z13 et Z16 avec une variation du nombre de copies est classifiée Grade I ;

le caractère bénin/malin de la tumeur thyroïdienne est diagnostiqué comme cancer thyroïdien potentiel lorsque les quantités exprimées d'au moins deux des gènes soumis à empreinte Z1, Z3, Z4, Z5, Z6, Z8, Z10, Z11, Z13 et Z16 avec une perte d'empreinte sont classifiées Grade I ; ou lorsque les quantités exprimées d'au moins deux des gènes soumis à empreinte Z1, Z3, Z4, Z5, Z6, Z8, Z10, Z11, Z13 et Z16 avec une variation du nombre de copies sont classifiées Grade I ; ou lorsque la quantité exprimée de pas plus d'un des gènes soumis à empreinte Z1, Z3, Z4, Z5, Z6, Z8, Z10, Z11, Z13 et Z16 avec une perte d'empreinte est classifiée Grade II, et que la quantité exprimée de pas plus d'un des gènes soumis à empreinte Z1, Z3, Z4, Z5, Z6, Z8, Z10, Z11, Z13 et Z16 avec une variation du nombre de copies est classifiée Grade II ; le caractère bénin/malin de la tumeur thyroïdienne est diagnostiqué comme cancer thyroïdien de stade précoce lorsque les quantités exprimées d'au moins deux des gènes soumis à empreinte Z1, Z3, Z4, Z5, Z6, Z8, Z10, Z11, Z13 et Z16 avec une perte d'empreinte sont classifiées Grade II ; ou lorsque les quantités exprimées d'au moins deux des gènes soumis à empreinte Z1, Z3, Z4, Z5, Z6, Z8, Z10, Z11, Z13 et Z16 avec une variation du nombre de copies sont classifiées Grade II ; ou lorsque la quantité exprimée de pas plus d'un des gènes soumis à empreinte Z1, Z3, Z4, Z5, Z6, Z8, Z10, Z11, Z13 et Z16 avec une perte d'empreinte est classifiée Grade III, et que la quantité exprimée de pas plus d'un des gènes soumis à empreinte Z1, Z3, Z4, Z5, Z6, Z8, Z10, Z11, Z13 et Z16 avec une variation du nombre de copies est classifiée Grade III ; le caractère bénin/malin de la tumeur thyroïdienne est diagnostiqué comme cancer thyroïdien de stade intermédiaire lorsque les quantités exprimées d'au moins deux des gènes soumis à empreinte Z1, Z3, Z4, Z5, Z6, Z8, Z10, Z11, Z13 et Z16 avec une perte d'empreinte sont classifiées Grade III ; ou lorsque les quantités exprimées d'au moins deux des gènes soumis à empreinte Z1, Z3, Z4, Z5, Z6, Z8, Z10, Z11, Z13 et Z16 avec une variation du nombre de copies sont classifiées Grade III ; ou lorsque la quantité exprimée de pas plus d'un des gènes soumis à empreinte Z1, Z3, Z4, Z5, Z6, Z8, Z10, Z11, Z13 et Z16 avec une perte d'empreinte est classifiée Grade IV, et que la quantité exprimée de pas plus d'un des gènes soumis à empreinte Z1, Z3, Z4, Z5, Z6, Z8, Z10, Z11, Z13 et Z16 avec une variation du nombre de copies est classifiée Grade IV ; et

le caractère bénin/malin de la tumeur thyroïdienne est diagnostiqué comme cancer thyroïdien avancé lorsque les quantités exprimées d'au moins deux des gènes soumis à empreinte Z1, Z3, Z4, Z5, Z6, Z8, Z10, Z11, Z13 et Z16 avec une perte d'empreinte sont classifiées Grade IV ; ou lorsque les quantités exprimées d'au moins deux des gènes soumis à empreinte Z1, Z3, Z4, Z5, Z6, Z8, Z10, Z11, Z13 et Z16 avec une variation du nombre de copies sont classifiées Grade IV.

11. Utilisation du modèle selon l'une quelconque des revendications 1 à 5 et/ou du dispositif selon la revendication 6 dans la détection d'une tumeur thyroïdienne.

12. Utilisation selon la revendication 11, dans laquelle le caractère bénin/malin de la tumeur thyroïdienne est classifié comme l'un des suivants : tumeur bénigne, cancer thyroïdien potentiel, cancer thyroïdien de stade précoce, cancer thyroïdien de stade intermédiaire et cancer thyroïdien avancé.

13. Utilisation selon la revendication 11 ou 12, dans laquelle un caractère bénin/malin de la tumeur thyroïdienne est diagnostiqué comme tumeur bénigne lorsque les quantités exprimées de chacun des gènes soumis à empreinte Z1, Z3, Z4, Z5, Z6, Z8, Z10, Z11, Z13 et Z16 avec, respectivement, une perte d'empreinte et une variation du nombre de copies sont toutes deux classifiées Grade 0 ; ou lorsque la quantité exprimée de pas plus d'un des gènes soumis à empreinte Z1, Z3, Z4, Z5, Z6, Z8, Z10, Z11, Z13 et Z16 avec une perte d'empreinte est classifiée Grade I, et que la quantité exprimée de pas plus d'un des gènes soumis à empreinte Z1, Z3, Z4, Z5, Z6, Z8, Z10, Z11, Z13 et Z16 avec une variation du nombre de copies est classifiée Grade I ;

le caractère bénin/malin de la tumeur thyroïdienne est diagnostiqué comme cancer thyroïdien potentiel lorsque les quantités exprimées d'au moins deux des gènes soumis à empreinte Z1, Z3, Z4, Z5, Z6, Z8, Z10, Z11, Z13 et Z16 avec une perte d'empreinte sont classifiées Grade I ; ou lorsque les quantités exprimées d'au moins deux des gènes soumis à empreinte Z1, Z3, Z4, Z5, Z6, Z8, Z10, Z11, Z13 et Z16 avec une variation du nombre de copies sont classifiées Grade I ; ou lorsque la quantité exprimée de pas plus d'un des gènes soumis à empreinte Z1, Z3, Z4, Z5, Z6, Z8, Z10, Z11, Z13 et Z16 avec une perte d'empreinte est classifiée Grade II, et que la quantité exprimée de pas plus d'un des gènes soumis à empreinte Z1, Z3, Z4, Z5, Z6, Z8, Z10, Z11, Z13 et Z16 avec une variation du nombre de copies est classifiée Grade II ;
le caractère bénin/malin de la tumeur thyroïdienne est diagnostiqué comme cancer thyroïdien de stade précoce lorsque les quantités exprimées d'au moins deux des gènes soumis à empreinte Z1, Z3, Z4, Z5, Z6, Z8, Z10, Z11, Z13 et Z16 avec une perte d'empreinte sont classifiées Grade II ; ou lorsque les quantités exprimées d'au moins deux des gènes soumis à empreinte Z1, Z3, Z4, Z5, Z6, Z8, Z10, Z11, Z13 et Z16 avec une variation du nombre de copies sont classifiées Grade II ; ou lorsque la quantité exprimée de pas plus d'un des gènes soumis à empreinte Z1, Z3, Z4, Z5, Z6, Z8, Z10, Z11, Z13 et Z16 avec une perte d'empreinte est classifiée Grade III, et que la quantité exprimée de pas plus d'un des gènes soumis à empreinte Z1, Z3, Z4, Z5, Z6, Z8, Z10, Z11, Z13 et Z16 avec une variation du nombre de copies est classifiée Grade III ;
le caractère bénin/malin de la tumeur thyroïdienne est diagnostiqué comme cancer thyroïdien de stade intermédiaire lorsque les quantités exprimées d'au moins deux des gènes soumis à empreinte Z1, Z3, Z4, Z5, Z6, Z8, Z10, Z11, Z13 et Z16 avec une perte d'empreinte sont classifiées Grade III ; ou lorsque les quantités exprimées d'au moins deux des gènes soumis à empreinte Z1, Z3, Z4, Z5, Z6, Z8, Z10, Z11, Z13 et Z16 avec une variation du nombre de copies sont classifiées Grade III ; ou lorsque la quantité exprimée de pas plus d'un des gènes soumis à empreinte Z1, Z3, Z4, Z5, Z6, Z8, Z10, Z11, Z13 et Z16 avec une perte d'empreinte est classifiée Grade IV, et que la quantité exprimée de pas plus d'un des gènes soumis à empreinte Z1, Z3, Z4, Z5, Z6, Z8, Z10, Z11, Z13 et Z16 avec une variation du nombre de copies est classifiée Grade IV ; et
le caractère bénin/malin de la tumeur thyroïdienne est diagnostiqué comme cancer thyroïdien avancé lorsque les quantités exprimées d'au moins deux des gènes soumis à empreinte Z1, Z3, Z4, Z5, Z6, Z8, Z10, Z11, Z13 et Z16 avec une perte d'empreinte sont classifiées Grade IV ; ou lorsque les quantités exprimées d'au moins deux des gènes soumis à empreinte Z1, Z3, Z4, Z5, Z6, Z8, Z10, Z11, Z13 et Z16 avec une variation du nombre de copies sont classifiées Grade IV.

FIG. 1

Grade 0

FIG. 2(a)

Grade I

FIG. 2(b)

Grade II

FIG. 2(c)

Grade III

FIG. 2(d)

Grade IV

FIG. 2(e)

FIG. 3(a)

FIG. 3(b)

FIG. 3(c)

FIG. 3(d)

FIG. 3(e)

FIG. 3(f)

FIG. 4(a)

FIG. 4(b)

FIG. 4(c)

FIG. 4(d)

FIG. 4(e)

FIG. 4(f)

FIG. 4(g)

FIG. 4(h)

FIG. 4(i)

FIG. 4(j)

FIG. 5(a)

**Z11**

FIG. 5(b)

**Z16**

FIG. 5(c)

**Z3**

FIG. 5(d)

**Z4**

FIG. 5(e)

**Z5**

FIG. 5(f)

**Z6**

FIG. 5(g)

FIG. 5(h)

FIG. 5(i)

FIG. 5(j)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2007015993 A1 **[0005]**
- WO 2016115530 A **[0005]**